# EUROPEAN PATENT SPECIFICATION

(11) **EP 3 761 866 B1**
(45) Date of publication and mention of the grant of the patent: **06.05.2026**
(21) Application number: 19710390.6
(22) Date of filing: 08.03.2019
(51) Int. Cl.: A61B 5/021, A61B 5/024, A61B 5/11, A61B 5/00, A61B 5/02, A61B 5/145, A61B 5/1455, A61B 5/08

(54) **CARDIOVASCULAR HEALTH MONITORING**
ÜBERWACHUNG DER KARDIOVASKULÄREN GESUNDHEIT
SURVEILLANCE DE LA SANTÉ CARDIOVASCULAIRE

(30) Priority: 08.03.2018 GB 201803703; 24.04.2018 GB 201806659
(43) Date of publication of application: 13.01.2021
(73) Proprietor: Biostealth Limited, London, Greater London W12 7JN (GB)
(72) Inventor: GBATI, Israel Ninsaw, London W12 7JN (GB)
(74) Representative: Kramer, Dani
(86) International application number: PCT/EP2019/055934
(87) International publication number: WO 2019/170903

(56) References cited:
- US-A1- 2014 081 153
- US-A1- 2015 374 245
- US-A1- 2016 157 781
- US-A1- 2016 278 646
- US-A1- 2017 209 055

## Description

### Field of Invention

The present invention relates to cardiovascular health monitoring. More specifically, the invention relates to a photoplethysmographic cardiovascular monitoring apparatus. The invention further relates to a surface for monitoring cardiovascular health of humans and other animals. The invention further relates to a trackpad device for monitoring cardiovascular health of users.

### Background

Cardiovascular illnesses are the number one cause of death worldwide. This is primarily because early symptoms of cardiovascular conditions are hard to detect by human sensory means.

Because of this, people more prone to having cardiovascular conditions are required to visit the hospital regularly for checkups and are also required to actively monitor their cardiovascular health at home using some form of portable handheld devices.

However, there is a problem with compliance. For instance, in the United States, less than 20% of all people required by their doctors to check their blood pressure daily, check only twice a week.

Owing to this and other problems relating to convenience of time and avenue for continuous cardiovascular health monitoring, there is the need to be able to monitor cardiovascular health of patients or people in general completely non-invasively without the patient or person wearing a device, initiating the process or even thinking about it.

Trackpads are also sometimes called touchpads. Trackpads are mostly used with computing devices to enable interaction with the computing device. They often include a touch sensing surface mostly capacitive and or resistive where the trackpad is configured to detect the position and motion of a user's finger or fingers that are in contact with the touch sensing surface.

The prior art relevant to the present invention includes several disclosures in the field of cardiovascular monitoring using photoplethysmography (PPG) sensors. US20160278646A1 describes an opto-physiological sensor with a single light source and photodetector, focusing on optimal emitter-detector separation for PPG signal acquisition. US20170209055A1 discloses a wearable biometric monitor that employs multiple PPG sensors to measure arterial stiffness, utilizing inertial sensors for signal quality assessment. US20140081153A1 details a pulse data detecting apparatus with interleaved arrays of light-emitting and light-receiving elements, analyzing all sensor pairs to select optimal signals for pulse rate calculation. US20150374245A1 presents a PPG device with interleaved arrays and proximity detection to initiate operation, analyzing all emitter-detector pairs for cardiovascular information. US20160157781A1 describes a computing device with an electrode-based contact detection system and optional PPG sensors that are activated uniformly.

### Summary of the Invention

Aspects and embodiments of the present invention are set out in the appended claims. These and other aspects and embodiments of the invention are also described herein.

According to at least one aspect described herein, there is provided a cardiovascular monitoring apparatus, the apparatus comprising: a photoplethysmography (PPG) sensor, having a plurality of light emitting elements and a plurality of light sensing elements, for obtaining at least one PPG signal from a patient; a member for supporting the plurality of light emitting elements and the plurality of light sensing elements, wherein the light emitting elements and light sensing elements are arranged as respective arrays on the member, and wherein the arrays are arranged such that the array of light emitting elements are interleaved with the array of light sensing elements; and a controller comprising: a detection module for detecting objects proximate the plurality of light sensing elements thereby to determine a subset of the plurality of light sensing elements, and, subsequently to detecting objects, obtaining signals using the subset of the plurality of light sensing elements; a determination module for determining whether signals obtained by the subset of the plurality of light sensing elements are each suitable for deriving information relevant to a patient's cardiovascular system; and a processing module for processing a signal that is determined to be suitable for deriving information relevant to the patient's cardiovascular system to derive information relevant to the patient's cardiovascular system (preferably wherein the information is relevant to the patient's cardiovascular health). This may provide improved processing efficiency, in that an initial test of the usefulness of a particular signal is provided prior to full processing. This may be particularly useful where it is desired to pick from a plurality of possible signals (optionally from a plurality of possible sensors). The PPG sensor may further comprise a plurality of light sensing elements.

Preferably, the determination module is arranged to determine whether signals obtained by at least a subset of the plurality of light sensing elements are each suitable for deriving information relevant to a patient's cardiovascular system. This may allow all possible signals from light sensing elements within a subset (wherein the subset is determined by some initial step e.g. by whether an object is detected proximate each light sensing element) to be tested for suitability, which may thereby facilitate a subsequent selection between signals. Optionally, the apparatus comprises a selector for performing this function.

Preferably, responsive to the determination module determining that a signal obtained by a particular light sensing element is not suitable for deriving information relevant to a patient's cardiovascular system, the determination module is arranged to determine whether a signal obtained by a different light sensing element is suitable for deriving information relevant to a patient's cardiovascular system. This may allow signals from light sensing elements be tested for suitability until an appropriate signal is found, which may improve processing speed and efficiency.

Preferably, determining whether a signal obtained by a particular light sensing element is suitable for deriving information relevant to a patient's cardiovascular system comprises comparing the obtained signal against one or more criteria. The one or more criteria may comprise at least one criterion relating to the shape of the signal waveform. Optionally, the criterion relates to whether the shape of the waveform is indicative of a patient's pulse. Preferably, the comparing to the at least one criterion relating to the shape of the signal waveform is performed using at least one of: Cross-Correlation, Sign Comparison, Sum of Absolute Difference, Interval Matching, and/or Dynamic Time Warping (DTW), and machine learning. The one or more criteria may comprise at least one criterion relating to the frequency of the signal.

The comparing may comprise comparing at least one period of the signal, preferably exactly one period of the signal, to the one or more criteria. The cardiovascular monitoring apparatus may further comprise means for storing a sample of the signal for comparison with the one or more criteria.

The controller may further comprise a selection module for selecting at least one signal that is determined to be suitable for deriving information relevant to the patient's cardiovascular system for processing by the processing module; said selection being from a plurality of such signals.

Alternatively, the first signal determined to be suitable for deriving information relevant to the patient's cardiovascular system may be processed (or selected to be processed). The selection may be based on the comparison against the one or more criteria, optionally such that the signal of the best quality (or a plurality of signals) is selected.

The controller may further comprise a detection module for detecting objects proximate the at least one light sensing element. Preferably, detecting objects comprises detecting a parameter related to the ambient light received by the at least one light sensing element; and comparing the detected parameter to a threshold value. Preferably, the at least one light emitting element is adapted to emit light only when an object is detected proximate the at least one light sensing element. This may allow power to be saved, which may be particularly useful where the apparatus is not powered by mains electricity or another such fixed source of power. The determination module may be arranged to perform the determination on only those signals obtained by the at least one light sensing element in relation to which a proximate object is detected.

The processing module may terminate processing of the signal when the signal ceases to be suitable for deriving cardiovascular parameters. The cardiovascular monitoring apparatus may further comprise a conditioner circuit, wherein the processing module may process the signal digitally. The controller may be arranged to control the at least one light emitting element.

The information relevant to a patient's cardiovascular system may comprise one or more cardiovascular parameters, preferably wherein the cardiovascular parameters are at least one of: blood pressure; arterial compliance; blood oxygen; pulse rate; pulse rate variability; and respiratory rate.

The plurality of light sensing elements may be arranged in an array. The PPG sensor may further comprise a plurality of light emitting elements; and the apparatus may further comprise a member for supporting the plurality of light emitting elements and the plurality of light sensing elements; wherein the light emitting elements and light sensing elements are arranged as respective arrays on the member; and wherein the arrays are arranged such that the array of light emitting elements are interleaved with the array of light sensing elements.

According to another aspect described herein, there is also provided a cardiovascular monitoring apparatus, the apparatus comprising: a photoplethysmography (PPG) sensor, having a plurality of light emitting elements and a plurality of light sensing elements, for obtaining at least one PPG signal from a patient; and a member for supporting the plurality of light emitting elements and the plurality of light sensing elements; wherein the light emitting elements and light sensing elements are arranged as respective arrays on the member; and wherein the arrays are arranged such that the array of light emitting elements are interleaved with the array of light sensing elements. This arrangement may allow a PPG sensor to be provided over a wide area, such as a surface of an object which a user grips for use. The PPG sensor is preferably arranged to obtain a signal via reflectance of light from a body part of the user (rather than via transmission of light through the body part).

Preferably, the cardiovascular monitoring apparatus further comprises a controller comprising: a determination module for determining whether a signal obtained by a particular light sensing element is suitable for deriving information relevant to a patient's cardiovascular system; and a processing module for processing a signal that is determined to be suitable for deriving information relevant to the patient's cardiovascular system to derive information relevant to the patient's cardiovascular system.

Preferably, the apparatus further comprises a module for determining (and optionally storing) at least one location within the array of the light sensing element(s) which obtain(s) a signal that is determined to be suitable for deriving information relevant to a patient's cardiovascular system. Preferably, the apparatus further comprises a module for determining (and optionally storing) at least one location of at least one body part of the patient in relation to the array. This may involve comparing signals obtained via various different light detecting elements. Determining the location of a body part may assist in processing, in particular in determining which signals to use.

Preferably, the apparatus further comprises a module for determining movement of at least one body part of the patient in relation to the array; optionally wherein the at least one body part is at least one finger.

The processing module may be arranged to process only those signals that correspond to light sensing elements in relation to which no movement of the art least one body part is detected, preferably over a predetermined period of time.

Optionally the processing module may be arranged to process only those signals that correspond to light sensing elements in relation to which no movement of the body part is detected over a particular time period. This may allow the most stable signals to be selected for processing, thereby improving the process of selecting the most appropriate signal.

Preferably, at least one of the array of light emitting elements and the array of light sensing elements extend over an area of the member that is larger than 10 cm²; preferably larger than 15 cm²; and more preferably larger than 20 cm². The size of array used may be approximately the size of a human hand/palm or (preferably) larger.

The member may be a flexible plastic substrate. The member may be curved. Preferably, the array of light emitting elements is arranged in or on a first layer of the member and the array of light sensing elements is arranged in or on a second layer of the member. Optionally, one of the first layer and the second layer is arranged on top of the other. Optionally, at least one of the first layer and the second layer comprise(s) one or more apertures to allow light to pass therethrough. Optionally, one of the array of light emitting elements and the array of light sensing elements are formed of a light emitter or light sensor and the one or more apertures in the first layer and/or the second layer, wherein, in use, the light emitter or light sensor is provided underneath the layer having the one or more apertures (i.e. whereby the apertures are arranged on the upper layer). The other of the array of light emitting elements and the array of light sensing elements may be arranged in or on the layer having the one or more apertures.

The apparatus may be configurable to operate as an input device for allowing a user to control a computing device.

According to another aspect described herein, there is provided a consumer product incorporating the apparatus as aforementioned. Preferably, the at least one light sensing element of the consumer product incorporating the apparatus is provided on a part of the product which is gripped by a user for using the product. Preferably, the consumer product provides a plurality of light sensing elements, the plurality of light sensing elements being distributed across at least one surface of the product which is gripped by a user for using the product. Preferably, the consumer product is one of: a laptop trackpad; a mouse for a computer; an electric toothbrush; and a shoe insole. The consumer product may alternatively be trackpad for a computing device or a computing device, such as a laptop computer, wherein the apparatus may be provided on a user input device such as a trackpad.

According to another aspect described herein, there is provided a system comprising two or more separate cardiovascular monitoring apparatuses as aforementioned. Preferably, each of the separate cardiovascular monitoring apparatuses is connected to a network. Preferably, the system further comprises computing means connected to the network adapted to store information or the cardiovascular parameters obtained by each apparatus.

Optionally, the cardiovascular monitoring apparatus further comprises a signal criteria module adapted to determine the one or more criteria. Optionally, the cardiovascular monitoring apparatus further comprises means for verifying the presence of a PPG signal.

According to another aspect described herein, there in provided a method, using a photoplethysmography (PPG) sensor having at least one light emitting element and at least one light sensing element for obtaining at least one PPG signal from a patient, comprising the steps of: determining whether a signal obtained by a particular light sensing element is suitable for deriving information relevant to a patient's cardiovascular system; and processing a signal that is determined to be suitable for deriving information relevant to the patient's cardiovascular system to derive information relevant to the patient's cardiovascular system. Optionally, the method comprises the further step of providing a photoplethysmography (PPG) sensor having at least one light emitting element and at least one light sensing element for obtaining at least one PPG signal from a patient.

According to another aspect described herein, there is provided a system comprising: a photoplethysmography (PPG) sensor having at least one light emitting element and at least one light sensing element for obtaining at least one PPG signal from a patient; a non-transitory memory storing instructions; and one or more hardware processors coupled to the non-transitory memory and configured to execute the instructions from the non-transitory memory to cause the system to perform operations comprising: determining whether a signal obtained by a particular light sensing element is suitable for deriving information relevant to a patient's cardiovascular system; and processing a signal that is determined to be suitable for deriving information relevant to the patient's cardiovascular system to derive information relevant to the patient's cardiovascular system.

The present disclosure provides a cardiovascular health monitoring surface (i.e. a cardiovascular monitoring apparatus), it's system and operating method thereof, capable of monitoring a user's cardiovascular health when the user touches (comes into contact with) the surface.

In this document the terms user, patient and person are used interchangeably, and they may mean any human or animal interacting with the invention. The phrases photoplethymograpical signal, photoplethysmogram signal, photoplethymopgrphy signal are also used interchangeably to mean an optically obtained plethysmogram obtained by illuminating the skin and measuring changes in light absorption.

In general aspect, a surface includes a light sensor (i.e. light sensing element) and a light source (i.e. light emitting element) with operably matching wavelength operably coupled to the surface and at least one controller operably coupled to the light sensor and light source.

The light sensor may be a photodiode, a phototransistor, a light dependent resistor, a light to frequency converter or any other sensor or transducer which shows changes in output due to changes in amount of light available at the sensing part of the sensor or transducer.

Implementation may include one or more of the following, for instance the at least one controller and the light sensor may be configured to detect the locations of one or more objects on (above) the surface (i.e. a detection module). The at least one controller and the light sensor may be configured to verify if the object or objects at a particular location on (above) the surface provides a photoplethysmography (PPG) signal. The at least one controller and the light sensor may also be configured to select at least one location with relatively better PPG signal compared to the other locations for further processing and extraction of cardiovascular indices (i.e. information relevant to a patient's cardiovascular system) such as blood pressure, blood oxygen, arterial compliance, pulse rate, respiratory rate, pulse rate variability and many more.

The at least one controller and the light sensor may detect the location or locations of one or more objects on (above) the surface by measuring the amount of light reflected back to the light sensor due to the placement of the object on (above) the light source. The intensity of the reflected light varies with the proximity of the object to the light sensor and light source, this may allow the location of the object to be precisely found. The object or objects locations may also be found by reading the amount of ambient light detected at the different locations of the cardiovascular surface.

The at least one controller and the light sensor may verify the presence and locations of PPG signal by sampling at least one period long signal from each location where an object is detected and then running each at least one period long signal through either a software or hardware based waveform matching and rejection criteria module (i.e. a determination module) which may be part of the at least one controller or as a standalone module.

The surface may be divided into plurality of regions (i.e. a subset of the plurality of light sensors) and the at least one controller may be configured to determine the location or locations of the one or more objects on (above) the surface by using the intensity of the reflected light detected by the light sensor at each region to filter through regions to find the object or objects locations.

The light sensor may be a matrix (i.e. an array) of light sensors. The light source may also be a matrix (i.e. an array) of light sources. The light source and light sensor may also form light-source/ light-sensor pairs (i.e. the array or light sources is interleaved with the array of light sensors). These pairs in turn may form a matrix of light-source/light-sensor pairs.

In another general aspect, a surface is made up of at least two layers, with a light sensor operably coupled to the top layer and a light source with operably matching wavelength coupled to the bottom layer and at least one controller operably coupled to the light sensor and light source.

The light sensor may be a photodiode, a phototransistor, a light dependent resistor, a light to frequency converter or any other sensor or transducer which show changes in output due to changes in amount of light available at the sensing part of the sensor or transducer.

Implementation may include one or more of the following, for example, the top layer operably coupled to the light sensor may be perforated so that the light source coupled to bottom layer may be able to illuminate an object on the top layer. The at least one controller and the light sensor may be configured to detect the location or locations of one or more objects on (above) the surface. The at least one controller and the light sensor may be configured to verify if the object or objects at a particular location on (above) the surface provides a photoplethysmography (PPG) signal. The at least one controller and the light sensor may also be configured to select at least one location with relatively better PPG signal compared to the other locations for further processing and extraction of cardiovascular indices (i.e. by a processing module) such as blood pressure, blood oxygen, arterial compliance, pulse rate, respiratory rate, pulse rate variability and many more.

The at least one controller and the light sensor may detect the location of one or more objects on (above) the surface by measuring the amount of light reflected back to the light sensor due to the placement of the object on (above) the light source. The intensity of the reflected light varies with the proximity of the object to the light sensor and light source; this may allow the location of the object to be precisely found.

The at least one controller and the light sensor may verify the presence and location of PPG signal by sampling at least one period long signal from each location where an object is detected and then running each at least one period long signal through either a software or hardware based waveform matching and rejection criteria module which may be part of the at least one controller or as a standalone module.

The surface may be divided into plurality of regions and the at least one controller may be configured to determine the locations of one or more objects on (above) the surface by using the intensity of the reflected light detected by the light sensor at each region to filter through regions to find the object or objects locations.

The light sensor may be a matrix of light sensors. The light source may also be a matrix of light sources. The light source and light sensor may also form light-source/ light-sensor pairs. These pairs in turn may form a matrix of light-source/light-sensor pairs.

In another general aspect, a surface is made up of at least two layers, with the light source operably coupled to the top layer and the light sensor of operably matching wavelength coupled to the bottom layer and at least one controller operably coupled to the light sensor and light source.

The light sensor may be a photodiode, a phototransistor, a light dependent resistor, a light to frequency converter or any other sensor or transducer which show changes in output due to changes in amount of light available at the sensing part of the sensor or transducer.

Implementation may include one or more of the following, for example, the top layer operably coupled to the light source may be perforated so that the light sensor operably coupled to bottom layer may be able to detect reflected rays (beams) of light coming from the object on the top layer. The at least one controller and the light sensor may be configured to detect the location or locations of one or more objects on (above) the surface. The at least one controller and the light sensor may be configured to verify if the object or objects at a particular location on (above) the surface provides a photoplethysmography (PPG) signal. The at least one controller and the light sensor may also be configured to select at least one location with relatively better PPG signal compared to the other locations for further processing and extraction of cardiovascular indices such as blood pressure, blood oxygen, arterial compliance, pulse rate, respiratory rate, pulse rate variability and many more.

The present disclosure also provides a cardiovascular health monitoring trackpad (or another consumer product), its system and operating method thereof, capable of monitoring user's cardiovascular health.

In general aspect a trackpad includes a top surface with a light sensor and a light source with operably matching wavelengths operably coupled to the top surface and at least one controller operably coupled to only the light sensor or the light sensor and the light source. The at least one controller and the light sensor are configured to detect one or more fingers on the top surface. The at least one controller is configured to determine the locations and movements of the one or more fingers on the top surface (i.e. a module for determining movement of a body part of the patient in relation to the array) using information from the detection by the at least one controller and the sensor. The at least one controller and the light sensor are also configured to select at least one finger location for photoplethysmopgraphy signal processing and extraction of cardiovascular parameters (i.e. information relevant to a patient's cardiovascular system) such as blood pressure, blood oxygen, arterial compliance, pulse rate, pulse rate variability and many others.

The light sensor may be a photodiode, a phototransistor, a light dependent resistor, a light to frequency converter or any other sensor or transducer which shows changes in output due to changes in amount of light available at the sensing part of the sensor or transducer.

Implementation may include one or more of the following, for instance, the top surface may be divided into plurality of regions and the at least one controller may be configured to determine the locations and movements of one or more fingers on the top surface by using the intensity of the reflected light detected by the light sensor at different regions. The intensity of the reflected light varies with the proximity of the finger to the light sensor and light source, this may allow the locations of the finger or fingers to be precisely measured. The finger or fingers locations may also be measured by measuring the amount of ambient light received by the light sensor at different regions of the top surface.

The at least one controller and the light sensor may select at least one finger location for processing the photoplethysmography (PPG) signal at that location or locations and extraction of cardiovascular parameters after the at least one controller has detected no finger movement for a set amount of time.

The light sensor may be a matrix of light sensors. The light source may be a matrix of light sources. The light sensor and light source may form a light-source/ light-sensor pair. These pairs may in turn form a matrix of light-source/light-sensor pairs.

The trackpad apparatus may also include the trackpad and the keyboard area existing as one entity and the at least one controller operably coupled to the light sensor may be used to switch between keyboard and trackpad mode by rapidly examining the pattern generated by the movement of the finger or fingers on the top surface.

In another general aspect a computing device includes a display device, where the computing device is configured to render graphical user interface (GUI) on the display device. The computing device includes a trackpad device configured to facilitate user interaction with the graphical user interface (GUI) of the computing device and interaction with computer software and applications. Examples of such interactions may include but not limited to the following, pointer (cursor) control, volume control, media control, scroll control, power control and all other operating systems, applications software, web, virtual and augmented reality controls available to computing systems including all Human-Interface Device (HID) control commands available to computing systems and devices.

The trackpad may include a top surface with a light sensor and a light source with operably matching wavelengths operably coupled to the top surface and at least one controller operably coupled to only the light sensor or the light sensor and the light source. The at least one controller and the light sensor are configured to detect one or more fingers on the top surface. The at least one controller is configured to determine the locations and movements of the one or more fingers on the top surface using information from the detection by the at least one controller and the sensor. The at least one controller and the light sensor are also configured to select at least one finger location for photoplethysmography (PPG) signal processing and extraction of cardiovascular parameters such as blood pressure, blood oxygen, arterial compliance, pulse rate, pulse rate variability and many others.

The light sensor may be a photodiode, a phototransistor, a light dependent resistor, light to wavelength converter and all other sensors and transducers which show changes in output due to changes in amount of light available at the sensing part of the sensor or transducer.

Implementation may include one or more of the following, for instance, the top surface of the trackpad may be divided into plurality of regions and the at least one controller may be configured to determine the locations and movements of one or more fingers on the top surface by using the intensity of the reflected light detected by the light sensor at different regions. The intensity of the reflected light varies with the proximity of the finger to the light sensor and light source, this may allow the locations of the finger or fingers to be precisely measured. The finger or fingers locations may also be measured by measuring the amount of ambient light received by the light sensor at different regions of the top surface.

The at least one controller and the light sensor of the trackpad apparatus may select at least one finger location for processing the photoplethysmography (PPG) signal at that location or locations and extraction of cardiovascular parameters after the at least one controller has detected no finger movement for a set amount of time.

The light sensor of the trackpad apparatus may be a matrix of light sensors. The light source of the trackpad apparatus may be a matrix of light sources. The light sensor and light source of the trackpad apparatus may form a light-source/ light-sensor pair. These pairs may in turn form a matrix of light-source/light-sensor pairs.

The trackpad apparatus may also include the trackpad and the keyboard area existing as one entity and the at least one controller operably coupled to the light sensor may be used to switch between keyboard and trackpad mode by rapidly examining the pattern generated by the movement of the finger or fingers on the top surface.

In another general aspect, a trackpad apparatus includes a top surface with at least two layers, with a light sensor operably coupled to the top layer and a light source of operably matching wavelength coupled to the bottom layer and at least one controller operably coupled to the light sensor or the light sensor and light source. The at least one controller and the light sensor are configured to detect one or more fingers on the top surface. The at least one controller is configured to determine the locations and movements of the one or more fingers on the top surface using information from the detection by the at least one controller and the sensor. The at least one controller and the light sensor are also configured to select at least one finger location for processing the photoplethysmography (PPG) signal at that location and extraction of cardiovascular indices such as blood pressure, blood oxygen, arterial compliance, pulse rate, pulse rate variability and many others.

Implementation may include one of the following, for example, the top layer operably coupled to the light sensor may be perforated so that the light source coupled to bottom layer may be able to illuminate a finger or fingers on the top layer. The top surface may be divided into plurality of regions and the at least one controller may be configured to determine the locations and movements of one or more fingers on the top surface by using the intensity of the reflected light detected by the light sensor at different regions. The intensity of the reflected light varies with the proximity of the finger to the light sensor and light source, this may allow the locations of the finger or fingers to be precisely measured. The finger or fingers locations may also be measured by measuring the amount of ambient light received by the light sensor at different regions of the top surface.

The at least one controller and the light sensor of the trackpad apparatus may select at least one finger location for processing the photoplethysmography (PPG) signal from that location or locations and extraction of cardiovascular indices after the at least one controller has detected no finger movement for a set amount of time.

The light sensor of the trackpad apparatus may be a matrix of light sensors. The light source of the trackpad apparatus may be a matrix of light sources. The light sensor and light source of the trackpad apparatus may form a light-source/ light-sensor pair. These pairs may in turn form a matrix of light-source/light-sensor pairs.

The trackpad apparatus may also include the trackpad and the keyboard area existing as one entity and the at least one controller operably coupled to the light sensor may be used to switch between keyboard and trackpad mode by rapidly examining the pattern generated by the movement of the finger or fingers on the top surface.

In another general aspect, a trackpad apparatus includes a top surface with at least two layers, with a light source operably coupled to the top layer and a light sensor of operably matching wavelength coupled to the bottom layer and at least one controller operably coupled to the light sensor or the light sensor and light source. The at least one controller and the light sensor are configured to detect one or more fingers on the top surface. The at least one controller is configured to determine the locations and movements of the one or more fingers on the top surface using information from the detection by the at least one controller and the sensor. The at least one controller and the light sensor are also configured to select at least one finger location for photoplethysmography (PPG) signal processing and extraction of cardiovascular parameters such as blood pressure, blood oxygen, arterial compliance, pulse rate, pulse rate variability and many others.

Implementation may include one of the following, for example, the top layer operably coupled to the light source may be perforated so that the light sensor coupled to bottom layer may be able to detect the reflected light ray or beam from a finger or fingers on the top layer. The top surface may be divided into plurality of regions and the at least one controller may be configured to determine the locations and movements of one or more fingers on the top surface by using the intensity of the reflected light detected by the light sensor at different regions. The intensity of the reflected light varies with the proximity of the finger to the light sensor and light source, this may allow the locations of the finger or fingers to be precisely measured. The finger or fingers locations may also be measured by measuring the amount of ambient light received by the light sensor at different regions of the top surface.

The at least one controller and the light sensor of the trackpad apparatus may select at least one finger location for processing the photoplethysmography (PPG) signal from that location or locations and extraction of cardiovascular parameter after the at least one controller has detected no finger movement for a set amount of time.

The light sensor of the trackpad apparatus may be a matrix of light sensors. The light source of the trackpad apparatus may also be a matrix of light sources. The light sensor and light source of the trackpad apparatus may form a light-source/ light-sensor pair. These pairs may in turn form a matrix of light-source/light-sensor pairs.

The trackpad apparatus may also include the trackpad and the keyboard area existing as one entity and the at least one controller operably coupled to the light sensor may be used to switch between keyboard and trackpad mode by rapidly examining the pattern generated by the movement of the finger or fingers on the top surface.

In general, improved cardiovascular health monitoring may be provided by monitoring the cardiovascular health of patients or persons through the objects (artifacts) they already interact with in their day-to-day activities, by transforming the surfaces of these object that come into contact with the user into a cardiovascular health monitoring surfaces.

In general, given the amount of time people spend each day using computers, computer trackpads can provide a good avenue for monitoring cardiovascular health. This can be achieved by designing trackpads in such a way that they can perform their core function which is to enable interaction with the computing device and also monitor the cardiovascular health of users.

The invention also provides a computer program or a computer program product for carrying out any of the processes described herein, and/or for embodying any of the apparatus features described herein, and a computer readable medium having stored thereon a program for carrying out any of the methods described herein and/or for embodying any of the apparatus features described herein.

The invention also provides a signal embodying a computer program or a computer program product for carrying out any of the methods described herein, and/or for embodying any of the apparatus features described herein, a method of transmitting such a signal, and a computer product having an operating system which supports a computer program for carrying out the processes described herein and/or for embodying any of the apparatus features described herein.

Any feature in one aspect of the invention may be applied to other aspects of the invention, in any appropriate combination. In particular, method aspects may be applied to apparatus aspects, and vice versa. As used herein, means plus function features may be expressed alternatively in terms of their corresponding structure, such as a suitably programmed processor and associated memory.

Furthermore, features implanted in hardware may generally be implemented in software, and vice versa. Any reference to software and hardware features herein should be construed accordingly.

As used herein, the term "cardiovascular monitoring" should preferably be understood to also encompass the concept of "hemodynamic monitoring".

As used herein, a reference to "light emitting elements" and "light sensing elements" in the plural preferably also connotes such elements in the plural, and vice versa.

As used herein, the term "cardiovascular surface" preferably connotes a PPG sensor arranged across a surface, for example a surface of an object, or a surface of member (which may be applied or affixed to an object).

As used herein, the term "interleaved" preferably connotes a condition in which first set of objects being placed between objects in a second set at regular intervals, more preferably such that objects in the first set alternate with objects in the second set (i.e, objects in the first set are arranged between, or are interstitial to, those in the second set, and vice versa).

The invention extends to methods, system and apparatus substantially as herein described and/or as illustrated with reference to the accompanying figures.

One or more aspects will now be described, by way of example only and with reference to the accompanying drawings having like-reference numerals, in which:
FIG.1 is a drawing illustrating the operation of a cardiovascular surface in accordance with an example implementation.
FIG.2 is a drawing illustrating the operation of a cardiovascular surface in accordance with an example implementation.
FIG. 3A is a drawing illustrating a cardiovascular surface in accordance with an example implementation
FIG. 3B is a drawing illustrating a flexible cardiovascular surface in accordance with an example implementation.
FIG. 3C is a drawing illustrating a cardiovascular surface in accordance with an example implementation.
FIG. 3D is a drawing illustrating a cardiovascular surface in accordance with an example implementation.
FIG. 4A is a block diagram illustrating a cardiovascular surface apparatus in accordance with an example implementation.
FIG. 4B is a block diagram illustrating a cardiovascular surface apparatus in accordance with an example implementation.
FIG. 4C is a block diagram illustrating a cardiovascular surface apparatus in accordance with an example implementation.
FIG. 4D is a block diagram illustrating a cardiovascular surface apparatus in accordance with an example implementation.
FIG. 4E is a block diagram illustrating a cardiovascular surface apparatus in accordance with an example implementation.
FIG. 5A is a diagram illustrating waveform matching and rejection criteria module in accordance with an example implementation.
FIG. 5B is a diagram illustrating waveform matching and rejection criteria module in accordance with an example implementation.
FIG. 6 is an example flow diagram illustrating example operations of the cardiovascular surface apparatus of FIGS. 3A,3B,3C,3D,4A,4B,4C,4D and 4E as implemented in example embodiments of FIGS. 1 and 2.
FIG. 7 is an example flow diagram illustrating example operations of the cardiovascular monitoring apparatus.
FIG. 8 is a block diagram of a computer system suitable for implementing one or more embodiments of the present disclosure.
FIG. 9A is a drawing illustrating a computing device in accordance with an example implementation.
FIG. 9B is a drawing illustrating a computing device in accordance with an example implementation.
FIG. 10 is a drawing illustrating the operation of a cardiovascular health monitoring trackpad apparatus in accordance with an example implementation
FIG. 11 is a diagram illustrating a cardiovascular health monitoring trackpad apparatus in accordance with an example implementation.
FIG. 12A is a diagram illustrating a cardiovascular health monitoring trackpad apparatus in accordance with an example implementation.
FIG. 12B is a diagram illustrating a cardiovascular health monitoring trackpad apparatus in accordance with an example implementation.
FIG. 13A is a block diagram illustrating a cardiovascular health monitoring trackpad apparatus in accordance with an example implementation.
FIG. 13B is a block diagram illustrating a cardiovascular health monitoring trackpad apparatus in accordance with an example implementation.
FIG. 14A is a block diagram illustrating a cardiovascular health monitoring trackpad apparatus in accordance with an example implementation.
FIG. 14B is a block diagram illustrating a cardiovascular health monitoring trackpad apparatus in accordance with an example implementation.
FIG. 15 is an example flow diagram illustrating example operations of the cardiovascular health monitoring trackpad apparatus.
FIG. 16 to 19 are examples of consumer products incorporating the cardiovascular monitoring apparatus.

### Detailed description

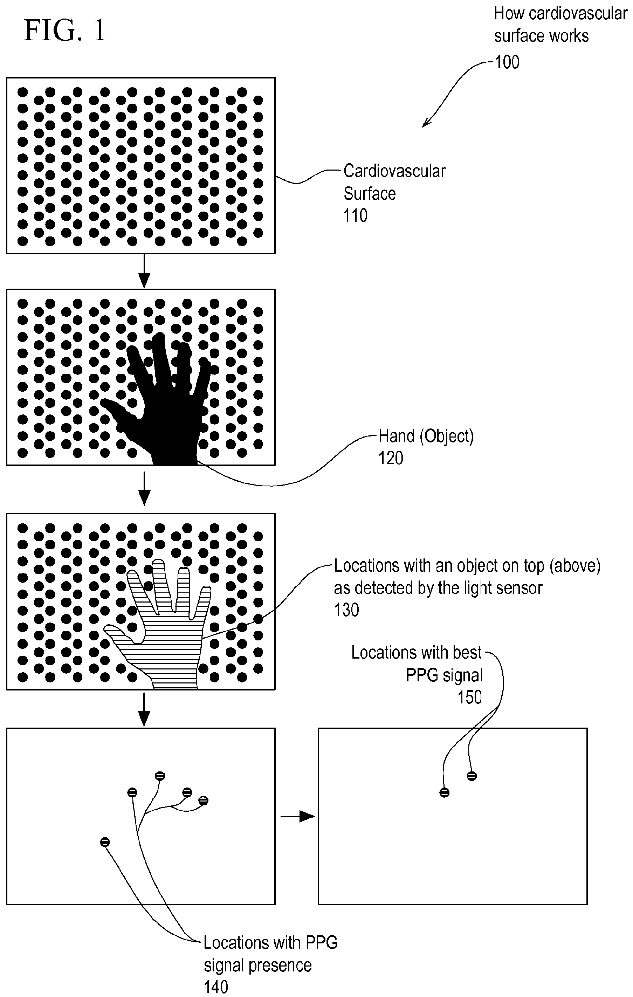

FIG. 1 is a drawing illustrating the operation of a cardiovascular surface in accordance with an example implementation. It will be appreciated that the cardiovascular surface here is shown by example and for the purpose of illustration.

The illustration 100 shown in FIG. 1 includes a hand 120 interacting with the cardiovascular surface 110. As indicated, when the hand 120 is placed on (above) the cardiovascular surface 110, the cardiovascular surface apparatus selects the locations of the cardiovascular surface where the object (hand) is placed 130. The cardiovascular surface apparatus proceeds to select the locations of the cardiovascular surface where photoplethysmographical (PPG) signal is present 140. The cardiovascular surface apparatus finally selects at least one location with relatively better quality PPG signal 150 for further processing and extraction of cardiovascular parameters.

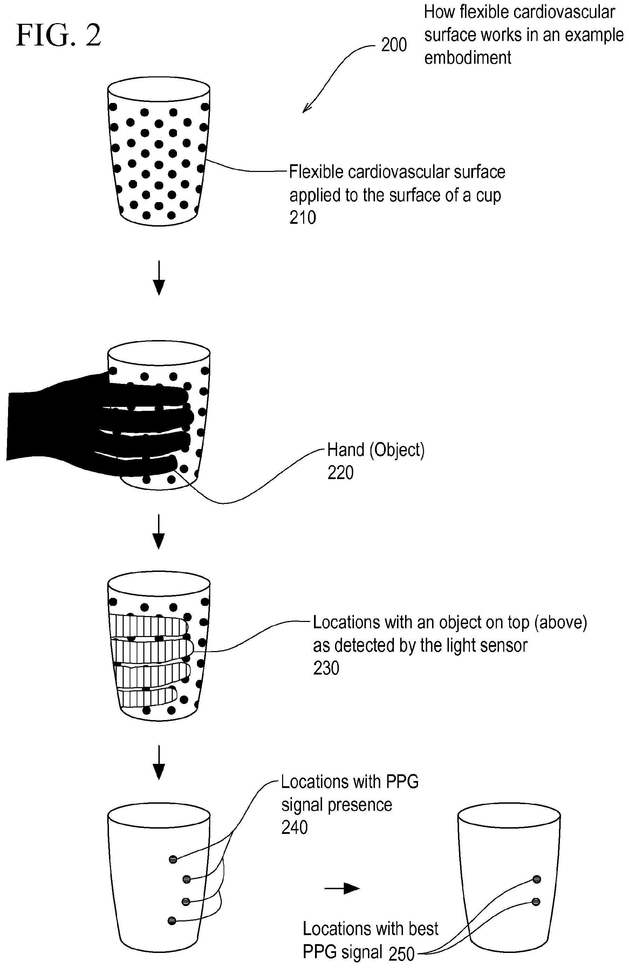

The illustration 200 shown in FIG. 2 includes a hand 220 interacting with a flexible cardiovascular surface 210 embedded into an example embodiment (cup). As indicated, when the hand 220 holds the cup, the cardiovascular surface apparatus selects the locations of the flexible cardiovascular surface where the hand is placed 230. The cardiovascular surface apparatus proceeds to select the locations of the embodiment (cup) where photoplethysmographical (PPG) signal is present 240. The cardiovascular surface apparatus finally selects at least one location with relatively better quality PPG signal 250 for further processing and extraction of cardiovascular parameters.

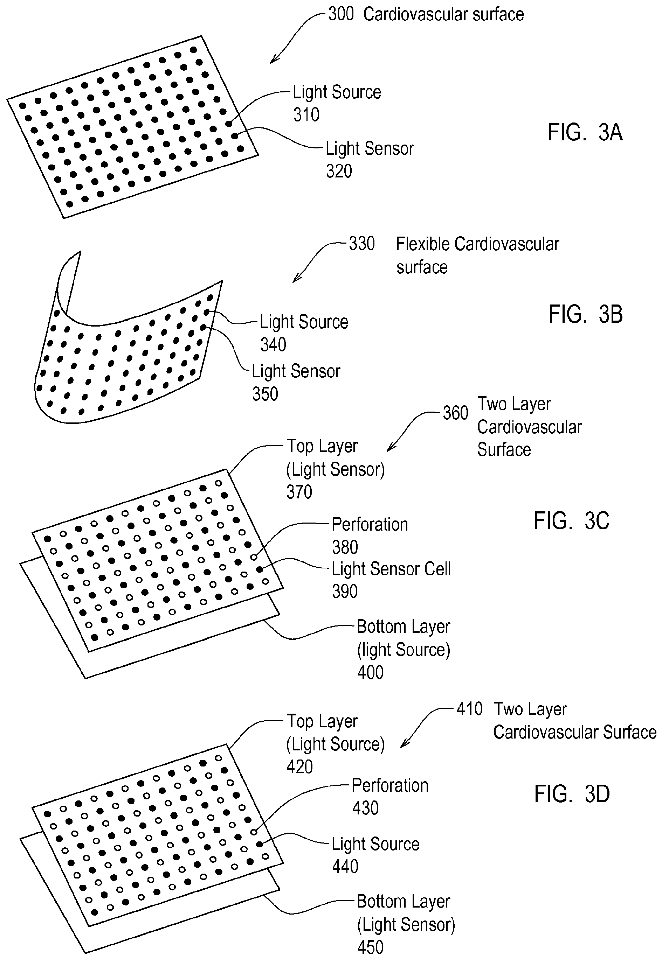

FIG. 3A is a drawing illustrating a cardiovascular surface in accordance with an example implementation. As indicated the cardiovascular surface 300 comprises a light source 310 and a light sensor 320.

FIG. 3B is a drawing illustrating a flexible cardiovascular surface in accordance with an example implementation. As indicated the flexible cardiovascular surface 330 comprises a light source 340 and a light sensor 350. The flexible cardiovascular surface may be made by mounting the light sensor 350 and the light source 340 onto a flexible circuit or a flexible plastic substrate (i.e. a member for supporting the plurality of light sources and light sensors) such as polymide, PEEK or transparent conductive polyester. The flexible cardiovascular surface 330 may be implemented in a number of ways, such as using the techniques described herein, for example. It will be appreciated that the particular configuration of the flexible cardiovascular surface 330 may vary and configuration used may depend on the specific implementation.

FIG. 3C is a drawing illustrating a two-layer cardiovascular surface in accordance with an example implementation. As indicated, the two-layer cardiovascular surface 360 comprises a top layer 370 which is operably coupled to the light sensor and a bottom layer 400 which is operably coupled to the light source. The top layer 370 also contains perforations 380, the perforations allow light from the bottom layer (light source) 400 to be able to illuminate an object on (above) the top surface 370. The light sensor may then be able to detect the amount of light reflected by the object on(above) the top surface.

FIG. 3D is a drawing illustrating a two-layer cardiovascular surface in accordance with an example implementation. As indicated, the two-layer cardiovascular surface 410 comprises a top layer 420 which is operably coupled to the light source and a bottom layer 450 which is operably coupled to the light sensor. The top layer 420 also contains perforations 430, the perforations allow the light sensor operably coupled to bottom layer to be able to detect reflected rays (beams) of light coming from an object on (above) the top layer.

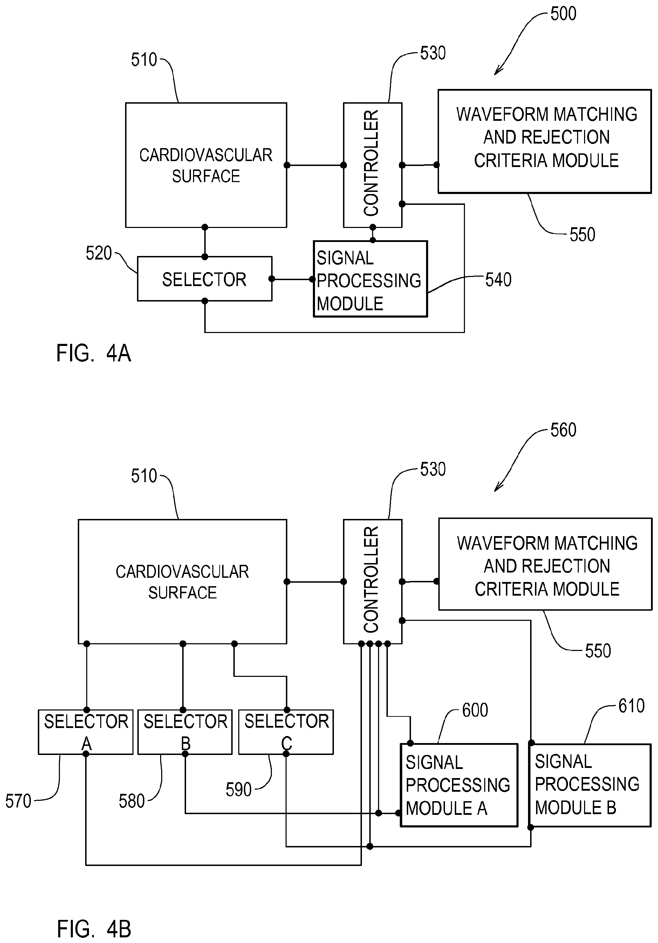

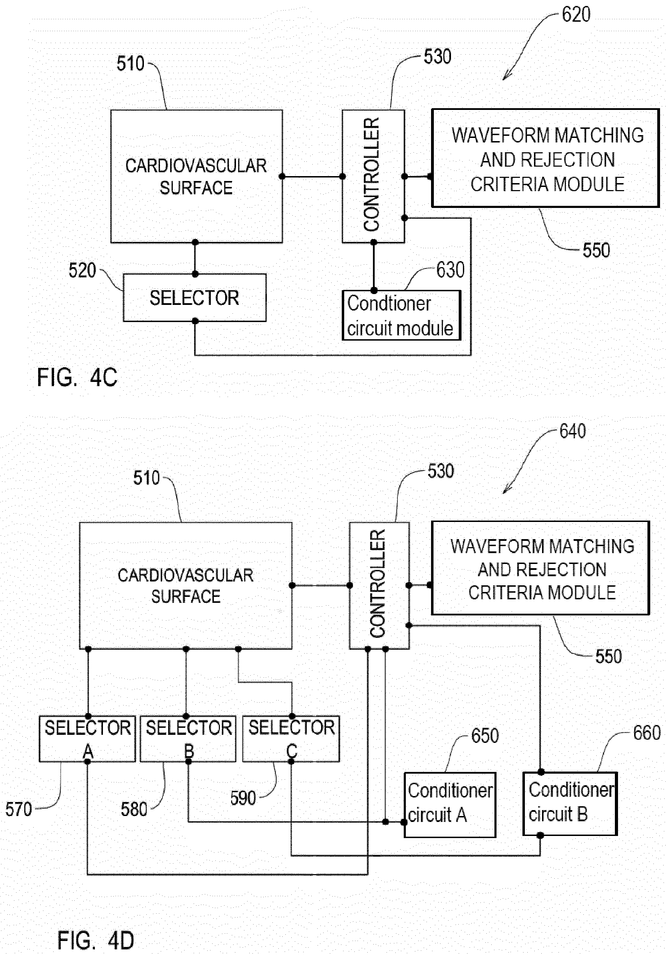

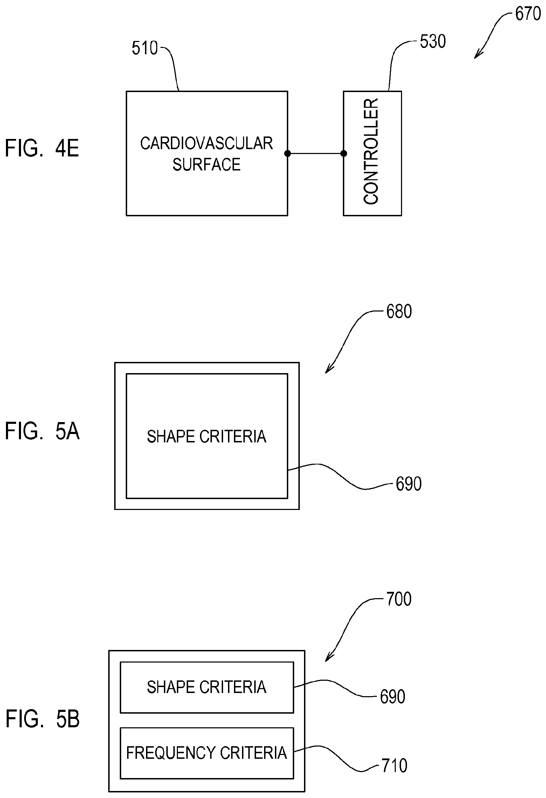

FIG. 4A is a block diagram illustrating the cardiovascular surface apparatus (system) 500 in accordance with an example implementation. The cardiovascular surface 510 may be implemented in rigid form like the rigid cardiovascular surfaces 300,360 and 410 or in a flexible form like the flexible cardiovascular surface 330. FIG. 4A illustrates a cardiovascular surface apparatus with a single selector 520, a single signal processing module 540, a single waveform matching and rejection criteria module 550 and a single controller 530. Of course the cardiovascular surface apparatus may be implemented in conjunction with other modules. For example, in other implementation more than one selector may be used. In yet another implementation the signal processing module 540 may be software based and a conditioner circuit such as a transimpedance amplifier or a voltage divider may be added to the system. For instance, as discussed below in more details in FIGS. 4B,4C, 4D and 4E.

In certain implementations, the cardiovascular surface apparatus 500 may include other elements or may be arranged in different fashions. In other instances, the Waveform Matching and Rejection Criteria module 550 may be included in the controller 530. In still another implementation, the function of the Waveform Matching and Rejection Criteria module such as described herein may be performed by other elements of the computing system (e.g. other than the controller 530) in which the cardiovascular surface apparatus 500 is implemented.

In an example implementation of the cardiovascular surface apparatus, the light source of the cardiovascular surface may be turned on when an object is on (above) the cardiovascular surface. The presence of an object may be detected by reading changes in the amount of ambient light received by the light sensor at different locations of the cardiovascular surface. This method may be one of the many ways the system may be able to operate to reduce power consumption. This method may ensure that the light source is only switched on when there is a reading to be made. Of course, other approaches for reducing power consumption are possible. It should be appreciated that this approach is given by way of example.

In another example implementation of the cardiovascular surface apparatus 500, the light source of the cardiovascular surface 510 may be turned on initially and may be used together with the light sensor to form a proximity sensor, such that the incident beam (ray) emitted by the light source at the area where an object is located is reflected by the object and received by the light sensor. This may be used to indicate (detect) the presence and location of an object on (above) the cardiovascular surface.

In another example implementation of the cardiovascular surface system/apparatus 500, the controller 530 may be used to control the light source of the cardiovascular surface. The controller may be configured to drive the light source of the cardiovascular surface by means of applying direct current or by means of pulse width modulation (PWM). The light source of the cardiovascular surface may be controlled by other methods as well, such as using different controller or applying direct current from a different source or using other digital or analog means.

The selector 520 and the controller 530 may store all locations of the cardiovascular surface 510 where an object is located (detected). An object is located (detected) by reading the changes in the amount of ambient light at all locations of the cardiovascular surface 510 or by using proximity thresholding.

When the locations of objects (or an object) on (or above) the cardiovascular surface 510 are identified, the selector 520, the signal processing module 540 and the controller 530 may proceed to collect at least one period long signal (waveform) from all the locations previously identified and stored by the selector 520 and the controller 530.

The cardiovascular surface apparatus (system) may use the Waveform Matching and Rejection Criteria module 550 to check whether each at least one period long signal (waveform) matches the criteria set by the Waveform Matching and Rejection Criteria module 550. The cardiovascular surface apparatus may eliminate (reject) the locations whose signal (waveform) do meet the criteria.

They cardiovascular surface apparatus(system) may select the location of at least one signal (waveform) with the closest match with the criteria set by the Waveform Matching and Rejection Criteria module 550 for continuous processing of signal from this location until the signal stops to meet the criteria set by the Waveform Matching and Rejection Criteria module.

The selector and the controller may select the first location of the cardiovascular surface where an object is located (detected), the signal processing module and the controller may proceed to collect at least one period long signal (waveform) from the selected location and then use the Waveform Matching and Rejection Criteria Module to check whether the at the at least one period long signal (waveform) from the selected location matches the criteria set by the Waveform Matching and Rejection Criteria module.

If the signal (waveform) matches the criteria set by the Waveform Matching and Rejection Criteria module (i.e. it is suitable for deriving information relevant to the patient's cardiovascular system) then the cardiovascular surface apparatus may proceed to select the location of the matched signal (waveform) for continuous processing of signal from that location until the signal no longer meets the criteria set by the Waveform Matching and Rejection Criteria module.

If the signal from the first selected location does not match the criteria set by the Waveform Matching and Rejection Criteria module, the selector and the controller may select the next location of the cardiovascular surface where an object was located (detected), the signal processing module and the controller may proceed to collect at least one period long signal from the selected location and then use the Waveform Matching and Rejection Criteria module to check if the selected signal meets the criteria set by the Waveform Matching and Rejection Criteria. This process continues until a location with a signal (waveform) which meets the criteria set by the Waveform Matching and Rejection Criteria module is found.

FIG. 4B is a block diagram illustrating the cardiovascular surface apparatus (system) 560 in accordance with an example implementation.

The cardiovascular surface 510 may be implemented in rigid form like the rigid cardiovascular surfaces 300,360 and 410 or in a flexible form like the flexible cardiovascular surface 330. FIG. 4B illustrates a cardiovascular surface apparatus (system) with a single controller 530. In other example implementations, more than one controller may be used. In yet another implementation, the signal processing module may be software based and a linear circuit module may be added to the system (apparatus).

As shown in FIG 4B, the cardiovascular surface apparatus 560 includes a cardiovascular surface 510, three selector modules; selectorA 570, selectorB 580 and selectorC 590 connected to the cardiovascular surface 510, a controller 530 connected to the cardiovascular surface 510 and the three selectors 570,580 and 590, two signal processing modules; signal processing moduleA 600 connected to selectorB 580 and controller 530 and signal processing moduleB 610 connected to selectorC 590 and controller 530. The cardiovascular surface apparatus(system) illustrated in FIG. 4B also includes a Waveform Matching and Rejection Criteria module 550 which is connected to controller 530.

In certain implementations, the cardiovascular surface apparatus 560 may include other elements or may be arranged in different fashions. In other instances, the Waveform Matching and Rejection Criteria module 550 may be included in the controller 530. In still another implementation, the function of the Waveform Matching and Rejection Criteria module such as described herein may be performed by other elements of the computing system (e.g. other than the controller 530) in which the cardiovascular surface apparatus 560 is implemented.

In an example implementation the controller 530 may be used to control the light source of the cardiovascular surface 510. The controller 530 may be configured to drive the light source of the cardiovascular surface by means of applying direct current or by means of pulse width modulation (PWM). The light source of the cardiovascular surface may be controlled by other methods as well, such as using a different controller or applying direct current from a different source or using other digital or analog means.

In an example implementation of the cardiovascular surface system/apparatus, the light source of the cardiovascular surface may be turned on when an object is on (above) the cardiovascular surface. The presence of an object may be detected by reading changes in the amount of ambient light received by the light sensor at different locations of the cardiovascular surface. This method may be one of the many ways the system may able to operate to reduce power consumption. This method may ensure that the light source is only switched on when there is a reading to be made. Of course, other approaches for reducing power consumption are possible. It should be appreciated that this approach is given by way of example.

In another example implementation of the cardiovascular surface apparatus 560, the light source of the cardiovascular surface 510 may be turned on initially and may be used together with the light sensor to form a proximity sensor, such that the incident beam (ray) emitted by the light source at the area where an object is located is reflected by the object and received by the light sensor. This may be used to indicate (detect) the presence and location of an object on (above) the cardiovascular surface.

SelectorA 570 and the controller 530 may store all locations of the cardiovascular surface 510 where an object is located (detected). Object detection may be done by looping through all locations of the cardiovascular surface and then using proximity or ambient light thresholding to identify the object (objects) locations.

SelectorB 580, controller 530 and signal processing moduleA 600 may collect at least one period long signal (waveform) from all the locations previously stored by selectorA 570 and the controller 530.

The cardiovascular surface apparatus (system) may use the Waveform Matching and Rejection Criteria module 550 to check whether each at least one period long signal (waveform) matches the criteria set by the Waveform Matching and Rejection Criteria module 550. The cardiovascular surface apparatus may eliminate (reject) the locations whose signal (waveform) do meet the criteria.

The cardiovascular surface apparatus(system) may select the location of at least one signal (waveform) with the closest match with the criteria set by the Waveform Matching and Rejection Criteria module 550 by using SelectorC 590 and the controller 530. SelectorC 590 (i.e. a selection module) may select the location of the signal(waveform) with the closest match with the criteria set by the Waveform Matching and Rejection Criteria module and connect that location to the signal processing moduleB 610 for continuous processing of signal from this location until the signal (waveform) from this location stops to meet the criteria set the Waveform Matching and Rejection Criteria module.

FIG. 4C is a block diagram illustrating the cardiovascular surface system(apparatus) 620 in accordance with an example embodiment. The cardiovascular surface 620 may be implemented in rigid form like the rigid cardiovascular surfaces 300,360 and 410 or in a flexible form like the flexible cardiovascular surface 330.

As shown in FIG. 4C, the cardiovascular surface system 620 includes a cardiovascular surface 510, a selector 520, a conditioner circuit 630 and a Waveform Matching and Rejection Criteria module 550. The conditioner circuit may be a transimpedance amplifier or a voltage divider. Of course the conditioner circuit can be constructed by other means also. It should be appreciated that the transimpedance amplifier circuit and the voltage divider circuit are stated as examples only.

In the cardiovascular surface system (apparatus) 620, the signal processing tasks are performed digitally by the controller 530 or by a separate controller which may be part of a different module of the cardiovascular surface system(apparatus).

In certain implementations, the cardiovascular surface apparatus (system) 620 may include other elements or may be arranged in different fashions. In other instances, the Waveform Matching and Rejection Criteria module 550 may be included in the controller 530. In still another implementation, the function of the Waveform Matching and Rejection Criteria module such as described herein may be performed by other elements of the computing system (e.g. other than the controller 530) in which the cardiovascular surface system 620 is implemented.

In an example implementation the controller 530 may be used to control the light source of the cardiovascular surface 620. The controller 530 may be configured to drive the light source of the cardiovascular surface by means of applying direct current or by means of pulse width modulation (PWM). The light source of the cardiovascular surface may be controlled by other methods as well, such as using a different controller or applying direct current from a different source or using other digital or analog means.

In an example implementation of the cardiovascular surface system, the light source of the cardiovascular surface may be turned on when an object is on (above) the cardiovascular surface. The presence of an object may be detected by reading changes in the amount of ambient light received by the light sensor at different locations of the cardiovascular surface. This method may be one of the many ways the system may able to operate to reduce power consumption. This method may ensure that the light source is only switched on when there is a reading to be made. Of course, other approaches for reducing power consumption are possible. It should be appreciated that this approach is given by way of example.

In another example implementation of the cardiovascular surface apparatus (system) 620, the light source of the cardiovascular surface 510 may be switched on initially and may be used together with the light sensor to form a proximity sensor, such that the incident beam (ray) emitted by the light source at the area where an object is located is reflected by the object and received by the light sensor. This may be used to indicate (detect) the presence and location of an object on (above) the cardiovascular surface.

The selector 520 and the controller 530 may store all locations of the cardiovascular surface 510 where an object is located (detected). Object detection may be done by looping through all locations of the cardiovascular surface and then using proximity or ambient light thresholding to identify the object (objects) locations.

When the locations of objects (an object) on (above) the cardiovascular surface 510 are identified, the selector 520, the conditioner circuit module 630 and the controller 530 may proceed to collect at least one period long signal (waveform) from all the locations previously identified and stored by the selector 520 and the controller 530.

The cardiovascular surface apparatus (system) may use the Waveform Matching and Rejection Criteria module 550 to check whether each at least one period long signal (waveform) matches the criteria set by the Waveform Matching and Rejection Criteria module 700. The cardiovascular surface apparatus may eliminate (reject) the locations whose signal (waveform) do meet the criteria.

The cardiovascular surface apparatus(system) may select the location of at least one signal (waveform) with the closest match with the criteria set by the Waveform Matching and Rejection Criteria module 550 for continuous processing of signal(waveform) from this location until the signal (waveform) from this location stops meeting the criteria set by the Waveform Matching and Rejection Criteria module.

FIG. 4D is a block diagram illustrating the cardiovascular surface apparatus 640 according to an example implementation.

The cardiovascular surface 510 may be implemented in rigid form like the rigid cardiovascular surfaces 300,360 and 410 or in a flexible form like the flexible cardiovascular surface 330. FIG. 4D illustrates a cardiovascular surface apparatus(system) with a single controller 530. In other example implementations, more than one controller may be used.

As shown in FIG 4D, the cardiovascular surface system(apparatus) 640 includes a cardiovascular surface 510, three selector modules; selectorA 570, selectorB 580 and selectorC 590 connected to the cardiovascular surface 510, a controller 530 connected to the cardiovascular surface 510 and the three selectors 570,580 and 590, two conditioner circuit modules; conditioner circuitA 650 connected to selectorB 580 and controller 530 and conditioner circuitB 660 connected to selectorC 590 and controller 530. The cardiovascular surface apparatus(system) illustrated in FIG. 4D also includes a Waveform Matching and Rejection Criteria module 550 which is connected to controller 530.

In certain implementations, the cardiovascular surface apparatus (system) 640 may include other elements or may be arranged in different fashions. In other instances, the Waveform Matching and Rejection Criteria module 550 may be included in the controller 530. In still another implementation, the function of the Waveform Matching and Rejection Criteria module such as described herein may be performed by other elements of the computing system (e.g. other than the controller 530) in which the cardiovascular surface system 640 is implemented.

In an example implementation the controller 530 may be used to control the light source of the cardiovascular surface 510. The controller 530 may be configured to drive the light source of the cardiovascular surface by means of applying direct current or by means of pulse width modulation (PWM). The light source of the cardiovascular surface may be controlled by other methods as well, such as using a different controller or applying direct current from a different source or using other digital or analog means.

In an example implementation of the cardiovascular surface system, the light source of the cardiovascular surface may be switched on when an object is on (above) the cardiovascular surface. The presence of an object may be detected by reading changes in the amount of ambient light received by the light sensor at different locations of the cardiovascular surface. This method may be one of the many ways the system may be able to operate to reduce power consumption. This method may ensure that the light source is only switched on when there is a reading to be made. Of course, other approaches for reducing power consumption are possible. It should be appreciated that this approach is given by way of example.

In another example implementation of the cardiovascular surface apparatus (system) 640, the light source of the cardiovascular surface 510 may be switched on initially and may be used together with the light sensor to form a proximity sensor such that the incident beam (ray) emitted by the light source at the area where an object is located is reflected by the object and received by the light sensor. This may be used to indicate (detect) the presence and location of an object on (above) the cardiovascular surface.

SelectorA 570 and the controller 530 may store all locations of the cardiovascular surface 510 where an object is located (detected). Object detection may be done by looping through all locations of the cardiovascular surface and then using proximity or ambient light thresholding to identify the object (objects) locations.

SelectorB 580, controller 530 and conditioner circuitA 650 may collect at least one period long signal (waveform) from all the locations previously stored by selectorA 570 and the controller 530 (i.e. means for storing a sample of the signal).

The cardiovascular surface apparatus (system) may use the pattern matching/rejection module 800 to check whether each at least one period long signal (waveform) meets the criteria set by the pattern matching/rejection module 800. The cardiovascular surface apparatus (system) may eliminate (reject) the locations whose signal (waveform) do not meet the criteria.

The cardiovascular surface apparatus(system) may select the location of at least one signal (waveform) with the closest match with the criteria set by the Waveform Matching and Rejection Criteria module 550. SelectorC 590 may select the location of the signal(waveform) with the closest match with the criteria set by the Waveform Matching and Rejection Criteria module and connect that location to conditioner circuitB 660 for continuous processing of signal from this location until the signal (waveform) from this location stops to meet the criteria set by the pattern matching/rejection module.

FIG. 4E is a block diagram illustrating a cardiovascular surface system (apparatus) 670 according to an example embodiment.

As indicated in FIG. 4E the cardiovascular surface system (apparatus) comprises a cardiovascular surface 510 and a controller 530. The cardiovascular surface system (apparatus) 670 may be designed to produce the same outcome as cardiovascular surface systems (apparatus) 500, 560,620 and 640.

In the cardiovascular surface system(apparatus) 670, the selector modules the Waveform Matching and Rejection Criteria modules and conditioner circuit modules or the signal processing modules as shown in cardiovascular surface system(apparatus) 500, 560,620 and 640 may be all contained in the controller 530.

FIG.5A is a diagram illustrating waveform matching and rejection (waveform filtering) criterial module 680 in accordance with an example implementation. In an example implementation, the waveform matching criteria 680 may be used to implement the waveform matching and rejection criteria module 550 shown in FIGS. 4A,4B,4C and 4D. Primarily, the waveform matching and rejection criteria module may be used to detect PPG waveforms for a cardiovascular surface using the techniques that have been described herein, such as with respect to FIGS. 4A,4B,4C and 4D. As indicated in FIG. 5A, the waveform matching and rejection criteria module may define acceptable and unacceptable waveforms.

As shown in FIG. 5A, the waveform matching and rejection criteria 680 includes shape criteria 690. In such an approach, the shape criteria 690 may define the shape of the waveform that should be accepted or the shape of the waveform that should be rejected. Of course other criteria may be used to implement the waveform matching and rejection criteria module for the cardiovascular surface apparatus.

The Waveform Matching/Rejection Criteria module may include at least a shape matching criteria. The shape matching criteria may be implemented by means of waveform matching and pattern recognition techniques where by a reference signal is compared to an incoming signal, where in this case the incoming signal may be the signals from the locations of the one or more objects on (above) the cardiovascular.

The waveform matching techniques that may be used may include Cross- Correlation, Sign Comparison, Sum of Absolute Difference, Interval Matching, Dynamic Time Warping (DTW) etc.

Of course, other waveform matching and pattern recognition techniques may be used to identify the PPG signal. It should be appreciated that these techniques are given by way example.

In the shape criteria approach the shape criteria may define the shape of the waveform that should be accepted or the shape of the waveform that should be rejected. Of course other criteria may be used to implement the Waveform Matching and Rejection Criteria for the cardiovascular surface apparatus.

Other approaches may include using machine learning and deep learning techniques to train a model that may be used to classify each at least one period signal from the location(s) of the cardiovascular surface where and object is located (detected) as acceptable or not acceptable for continuous processing of PPG signal and processing cardiovascular parameter.

Another example implementation is shown in FIG 5B. As shown in FIG. 5B, the waveform matching and rejection criteria module 700 comprises shape criteria 690 and frequency criteria 710. In such an approach, the shape 690 may define the shape of the waveform that should be accepted or the shape of the waveform that should be rejected. The frequency criteria 710 may define the frequency of the waveform (signal) that should be accepted or the frequency of the waveform that should be rejected.

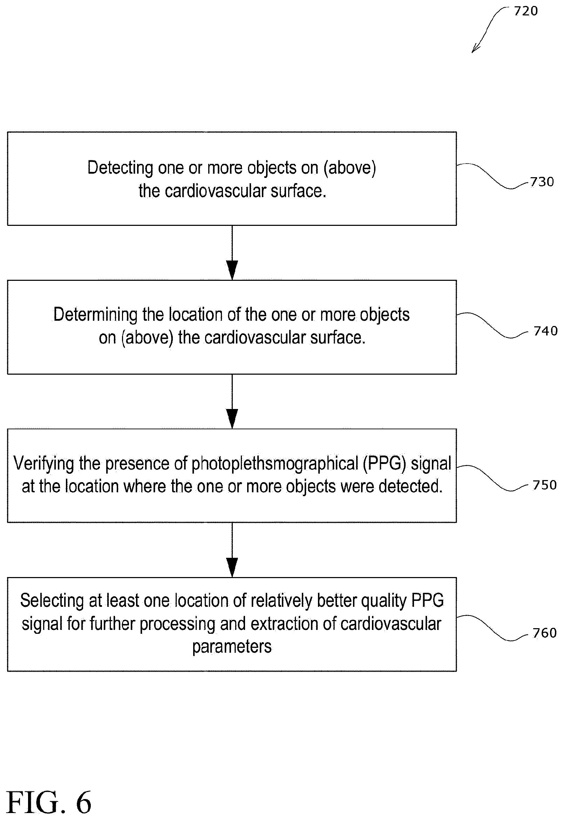

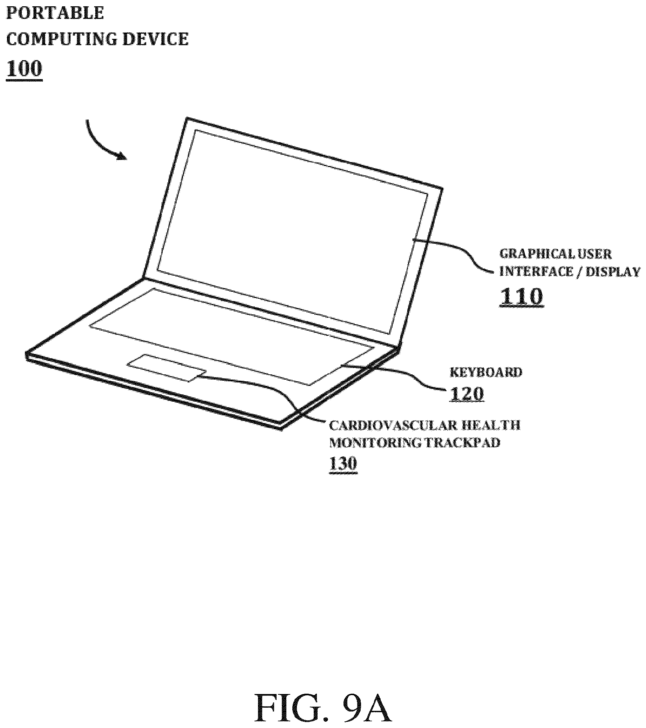

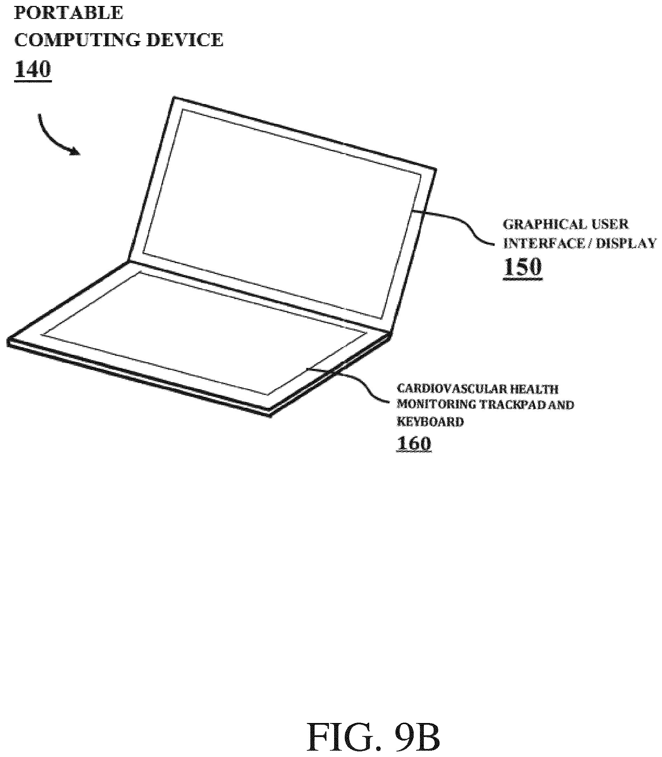

FIG. 6 is an example flow diagram illustrating the operation of cardiovascular surface apparatus of FIGS. 4A, 4B, 4C,4D and 4E. FIG.9 includes a process 720. The process 720 include detecting one or more objects on (above) the cardiovascular surface 730. For example, the controller may be configured to use ambient light thresholding or proximity thresholding may to detect one or more objects on (above) the cardiovascular surface.

Process 720 includes determining the locations of the one or more objects on (above) the cardiovascular surface 740. For example, the controller may be configured to use ambient light thresholding or proximity thresholding to determine the exact location or locations of one more objects on (above) the cardiovascular surface.

Process 720 also includes verifying the presence of photoplethysmographycal (PPG) signal at the locations where the one or more objects were detected 750. For example, the controller and Waveform Matching and Rejection Criteria module may be configured to either accept or reject waveforms(signals) from the locations where one or more objects is detected based on the criteria set base the by Waveform Matching and Rejection Criteria.

Process 720 ends with electing at one location of relatively better quality PPG signal for further processing and extraction of cardiovascular indices 760.

The controller of the cardiovascular surface apparatus described in this document may be implemented in a number of ways. The controller may be implemented using a general purpose programmable processor or controller. In other implementations, using an application specific integrated circuit which may include at least one processor. The controller may be implemented using a firmware/software in the form of machine readable instructions that may be executed by a general purpose processor or controller. The controller may also be implemented using a combination of techniques discussed above, or may be implemented using other techniques and/or devices.

The selector of the cardiovascular surface apparatus described in this document may be implemented in a number of ways. As an example, the selector may be implemented using mechanical switches, FETS or multiplexers.

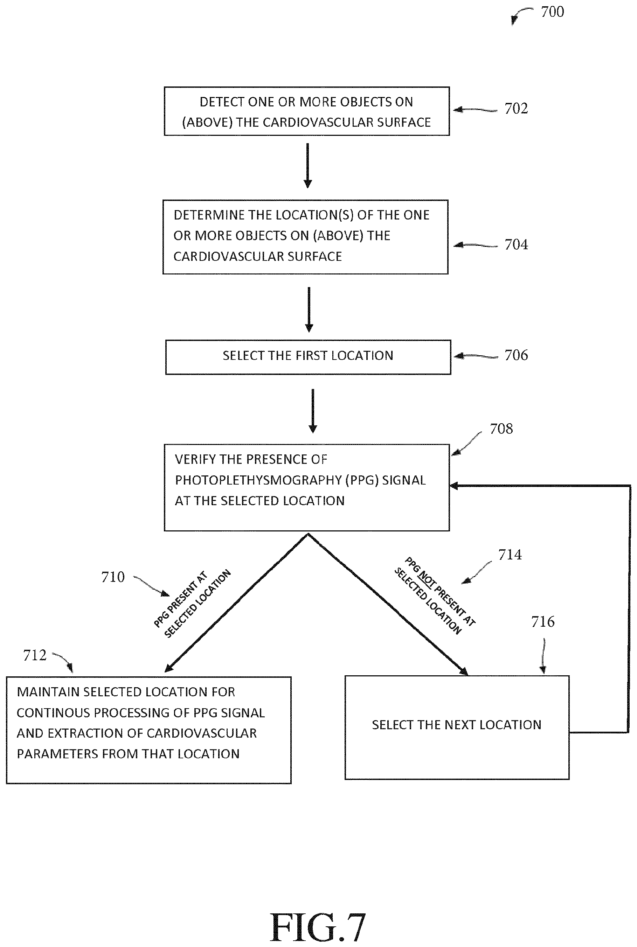

Figure 7 is an example flow diagram illustrating example operations of the cardiovascular monitoring apparatus. A function of the cardiovascular monitoring apparatus is to determine information, such as cardiovascular parameters, that are clinically useful. The parameters are extracted or calculated from the PPG signal obtained by the PPG sensor. If the signal obtained by the PPG sensor is low quality, the parameters calculated on the basis of it will be inaccurate and unreliable and therefore not clinically useful. This disclosure provides a method of operation of the cardiovascular monitoring apparatus that may ensure that parameters calculated from the PPG signal are reliable and clinically useful. This method of operation 700 is shown in the flow diagram of Figure 7.

As described, the cardiovascular apparatus typically has a plurality of light emitting elements and a plurality of light sensing elements. The light emitting elements are arranged in one plane, for example fixed in or on one sheet of material, and the light sensing elements are arranged in another plane, for example fixed in or on another sheet of material. The two sheets of material are arranged one on top of the other forming a cardiovascular sensing 'surface', where the light sensing elements are interleaved with / interposed between the light emitting elements, and vice versa. When a part of a patient is placed on or above the cardiovascular surface, the light emitted from the light emitters is reflected from that part of the patient so that it is received by the light sensors which obtain a PPG signal from the patient. Each light sensor is associated with a light emitter, or multiple light emitters, nearest to the sensor for obtaining a PPG signal.

At step 702 of the method 700, an object placed on or above the cardiovascular surface is detected. As described, the cardiovascular surface may detect objects placed on or above it by measuring the ambient light received by each of the plurality of light sensors. When an object is placed on or above the surface, the object will block at least some of the ambient light from reaching the light sensors and so those sensors which are blocked will measure a lower level of ambient light. A threshold in the level of the measured ambient light may be set, below which it is deemed that an object is on or above the surface. Alternatively or additionally, the apparatus may compare the ambient light received by each of the plurality of sensors. The apparatus may then determine that an object is on or above the surface when at least one of the sensors measure a lower ambient light level than other sensors (for example, lower by a threshold amount) and when the other sensors measure the same ambient light level as one another.

One advantage of first detecting whether there is an object above the surface before initiating the process of obtaining a PPG signal is a reduction in power consumption. The reduction is achieved by having the light emitting elements emit the light necessary to obtain a PPG signal only when it is determined that there is an object actually present above the sensor. In this way, the light emitters will only be turned on when necessary.

Once it is detected that one or more objects are on or above the cardiovascular surface at step 702, the location(s) of the one or more objects are determined at step 704. The locations of the objects are determined by identifying the locations of the particular light sensors which measure a lower ambient light level, as this low ambient light level measurement indicates that an object is on or above the location of those particular sensors. The light emitting elements and light sensing elements are typically set out as a grid as shown in the figures, therefore the location of the light sensors may be recorded as a coordinate on that grid for example.

Once the locations of the objects on or above the sensor have been determined, the cardiovascular monitoring apparatus selects one of those locations (i.e. selects the sensor at one of those locations) in step 706 as a 'first location'. The first location may be selected randomly from the locations above which it is determined that there is an object present. Alternatively, the first location may be selected based on the ambient light measurements made by each of the light sensors. In the latter case, it could be assumed that the sensor which will obtain the best PPG signal will be the sensor that is in closest contact with the body part of the patient, and therefore the sensor which measures the lowest level of ambient light. Therefore, the apparatus may select as the first location at step 706 the sensor which has the lowest ambient light reading.

At step 708, the presence of a PPG signal is verified at the selected location. It is possible that the object which is detected on or above the cardiovascular sensing surface is not part of a patient's body, or it is a part of a patient's body that is covered by clothing. In these cases and others, it would not be possible to obtain a PPG signal and therefore there would be no reason to continue attempting to obtain a PPG signal.

The verifying step 708 may include obtaining a signal from the sensor at the selected location for a pre-set amount of time and verifying the presence of a PPG waveform in the obtained signal. The pre-set amount of time may be equal to a small number of periods of a typical PPG signal, so as to allow enough time to collect a signal which contains at least one whole waveform that can be compared to the criteria while not collecting the signal for longer than it necessary to make the comparison.

If it is determined at step 708 that there is a PPG signal present at the selected location (step 710) then the selected location is maintained for continuous obtaining and processing of the PPG signal from that location (i.e. obtained by the light sensor(s) at that location) at step 712. The processing of the PPG signal at step 712 includes extracting cardiovascular parameters from the PPG signal obtained from the sensor(s) at the selected location.

If a PPG signal is found not to be present at the selected location at step 714, a next location at which it was determined (at step 704) that there was an object is selected at step 716. The next location may be selected at random from those locations or the next selected location may be selected based on the level of ambient light as previously described (i.e. the sensor measuring the second lowest level of ambient light).

Whilst the method 700 shows that, if it is found that the selected sensor is not measuring a PPG signal, the apparatus selects the next location and proceeds sequentially through the sensor locations, the step 708 could instead be performed simultaneously on each sensor. In this case, the measurements from each of the sensors that are determined to have an object above them (in step 704) are all checked to verify the presence of a PPG signal at any of those sensors. The apparatus then selects a first location from the sensors which are measuring a PPG signal and determines whether that signal is suitable for extracting cardiovascular information. If so, that sensor is continuously monitored. If not, another sensor that is measuring a PPG signal is selected.

Once the next location is selected at step 714, the method returns to step 708 to verify the presence of a PPG signal at the next location. The method will continue to iterate through all of the locations on or above which it was determined that there is an object (step 704). The iteration will continue either until a sensor location is found that provides an acceptable PPG signal (and then that signal is continuously processed) or until each of the sensor locations has been checked and no acceptable PPG signal has been detected (at which point the method will end).

In one example implementation, the cardiovascular monitoring apparatus is incorporated into a laptop trackpad, where the apparatus has a rectangular array of alternate light sensors and light emitters. In this example, the method 700 is carried out as follows.

A user of the laptop may place multiple fingers on the track pad. In the first step of the method the apparatus determines, by measuring the ambient light received by the light sensors, whether or not there are objects on or above the apparatus (the sensing surface). Once the apparatus has determined that there are objects above the surface, the apparatus determines the location of those objects by identifying which of the light sensors are obscured by the objects. In this example, the locations may be determined using an X-Y coordinate in the rectangular array of lights and sensors. Of all the identified sensor locations, a first location is selected. The light emitting element(s) that is associated with the selected sensor is then turned on so that the sensor can verify whether a PPG signal is present. If the sensor does measure a PPG signal, the sensor is continuously monitored and the signal is processed to obtain cardiovascular information (as described elsewhere, this step preferably also includes a verification that the PPG signal is actually suitable, for example in terms of the signal quality and consistency, for determining reliable and useful cardiovascular information on the basis of it). If the sensor does not measure a PPG signal, the apparatus selects the next sensor and repeats the process.

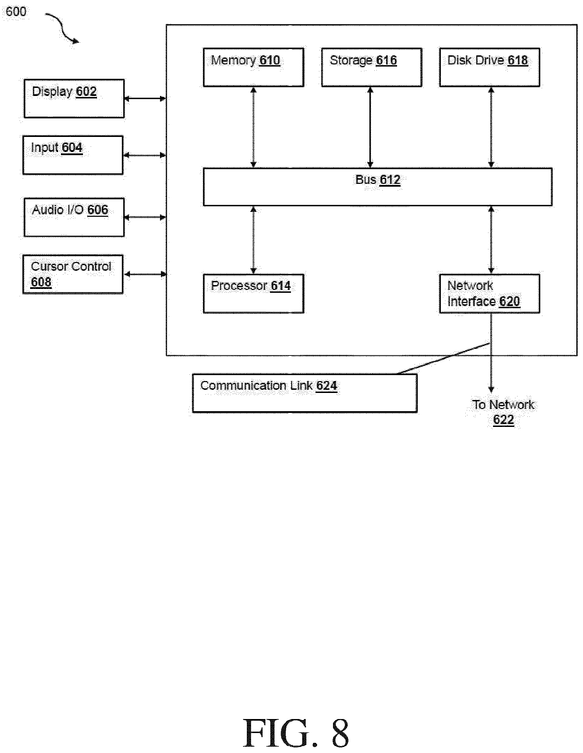

Figure 8 is a block diagram of a computer system 600 suitable for implementing one or more embodiments of the present disclosure, including the service provider server 130, the merchant server 120, and the user device 110. It should be appreciated that the apparatus described herein may be implemented as the computer system 600 in a manner as follows.

The computer system 600 includes a bus 612 or other communication mechanism for communicating information data, signals, and information between various components of the computer system 600. The components include an input/output (I/O) component 604 that processes a user (i.e., sender, recipient, service provider) action, such as selecting keys from a keypad/keyboard, selecting one or more buttons or links, etc., and/or receives an input from the PPG sensor, and sends a corresponding signal to the bus 612. The I/O component 604 may also include an output component, such as a display 602 and a cursor control 608 (such as a keyboard, keypad, mouse, etc.). The display 602 may be configured to present e.g. the output of the PPG sensor. An optional audio input/output component 606 may also be included to allow a user to use voice for inputting information by converting audio signals. The audio I/O component 606 may allow the user to hear audio. A transceiver or network interface 620 transmits and receives signals between the computer system 600 and other devices, such as the PPG sensor. In one embodiment, the transmission is wireless, although other transmission mediums and methods may also be suitable. A processor 614, which can be a micro-controller, digital signal processor (DSP), or other processing component, processes these various signals, such as for display on the computer system 600 or transmission to other devices via a communication link 624. The processor 614 may also control transmission of information, such as cookies or IP addresses, to other devices.

The components of the computer system 600 also include a system memory component 610 (e.g., RAM), a static storage component 616 (e.g., ROM), and/or a disk drive 618 (e.g., a solid-state drive, a hard drive). The computer system 600 performs specific operations by the processor 614 and other components by executing one or more sequences of instructions contained in the system memory component 610. For example, the processor 614 can perform the model generation functionalities described herein according to the process 400.

Logic may be encoded in a computer readable medium, which may refer to any medium that participates in providing instructions to the processor 614 for execution. Such a medium may take many forms, including but not limited to, non-volatile media, volatile media, and transmission media. In various implementations, non-volatile media includes optical or magnetic disks, volatile media includes dynamic memory, such as the system memory component 610, and transmission media includes coaxial cables, copper wire, and fiber optics, including wires that comprise the bus 612. In one embodiment, the logic is encoded in non-transitory computer readable medium. In one example, transmission media may take the form of acoustic or light waves, such as those generated during radio wave, optical, and infrared data communications.

Some common forms of computer readable media includes, for example, floppy disk, flexible disk, hard disk, magnetic tape, any other magnetic medium, CD-ROM, any other optical medium, punch cards, paper tape, any other physical medium with patterns of holes, RAM, PROM, EPROM, FLASH-EPROM, any other memory chip or cartridge, or any other medium from which a computer is adapted to read.

In various embodiments of the present disclosure, execution of instruction sequences to practice the present disclosure may be performed by the computer system 600. In various other embodiments of the present disclosure, a plurality of computer systems 600 coupled by the communication link 624 to the network (e.g., such as a LAN, WLAN, PTSN, and/or various other wired or wireless networks, including telecommunications, mobile, and cellular phone networks) may perform instruction sequences to practice the present disclosure in coordination with one another.

Where applicable, various embodiments provided by the present disclosure may be implemented using hardware, software, or combinations of hardware and software. Also, where applicable, the various hardware components and/or software components set forth herein may be combined into composite components comprising software, hardware, and/or both without departing from the spirit of the present disclosure. Where applicable, the various hardware components and/or software components set forth herein may be separated into sub-components comprising software, hardware, or both without departing from the scope of the present disclosure. In addition, where applicable, it is contemplated that software components may be implemented as hardware components and vice-versa.

Software in accordance with the present disclosure, such as program code and/or data, may be stored on one or more computer readable mediums. It is also contemplated that software identified herein may be implemented using one or more general purpose or specific purpose computers and/or computer systems, networked and/or otherwise. Where applicable, the ordering of various steps described herein may be changed, combined into composite steps, and/or separated into sub-steps to provide features described herein.

The various features and steps described herein may be implemented as systems comprising one or more memories storing various information described herein and one or more processors coupled to the one or more memories and a network, wherein the one or more processors are operable to perform steps as described herein, as non-transitory machine-readable medium comprising a plurality of machine-readable instructions which, when executed by one or more processors, are adapted to cause the one or more processors to perform a method comprising steps described herein, and methods performed by one or more devices, such as a hardware processor, user device, server, and other devices described herein.

FIG.9 is a drawing illustrating a computing device 100 in accordance with an example implementation. It will be appreciated that the computing device 100 is shown by way of example, and for purposes of illustration. In some implementations, the computing device 100 may have other configurations. For instance, the computing device 100 may be a tablet computer, a notebook computer, a desktop computer, a server computer or a number of other computing or electronics devices where a cardiovascular health monitoring trackpad apparatus 130, such as those described herein, may be used to facilitate interaction with corresponding device. Examples of such interactions may include but not limited to the following, pointer (cursor) control, volume control, media control, scroll control, power control and all other operating systems, applications software, web, virtual and augmented reality controls available to computing systems including all Human-Interface Device (HID) control commands available to computing systems and devices. Throughout this document the terms trackpad, trackpad device, trackpad apparatus, touchpad, touchpad device and touchpad apparatus may be used interchangeably. Also throughout this document, the terms computing device, computing system and electronic device may be used interchangeably. Also, in this document the terms user, patient and person are used interchangeably, and they may mean any human interacting with the invention. The phrases photoplethysmograpical signal, photoplethysmogram signal, photoplethysmopgraphy signal are also used interchangeably to mean an optically obtained plethysmogram obtained by illuminating the skin and measuring changes in light absorption.

The computing device 100 shown in FIG. 9 includes a display device 110, a keyboard 120, and a cardiovascular health monitoring trackpad 130. As indicated in FIG.9, the display device 110 may be configured to render a GUI that allows a user to interact with the computing device 100, such as to run programs, browse the internet, or draft documents, as some examples. A user of the computing device 100 may interact with the computing device 100 via the GUI rendered on the display device 110 using the keyboard 120, such as to enter text or commands. The keyboard 120 may take a number of forms, and particular arrangement of keyboard 120 will depend on the particular implantation.

A user may also interact with the computing device 100 via the GUI rendered on the display device 110 using the cardiovascular health monitoring trackpad 130, as to move cursor, select objects, launch programs from icons or move objects in the GUI, as some examples. Of course, other interactions with the GUI are possible using the cardiovascular health monitoring trackpad 130. The trackpad 130 may be implemented in a number of ways such as using the techniques described herein, for example. It will be appreciated that the particular configuration of the trackpad 130 may vary and the configuration used will depend on the specific implementation. For instance, the trackpad may be increased in size and disposed in (replace) the area that include the keyboard 130, such as described with respect to FIG.9B

FIG.9B is a drawing illustrating a computing device 140 in accordance with an example implementation. It will be appreciated that the computing device 140 is shown by way of example, and for purposes of illustration. In some implementations, the computing device 140 may have other configurations. For instance, the computing device 140 may be a tablet computer, a desktop computer, a server computer, or a number of other computing or electronics devices where a combined keyboard and cardiovascular health monitoring trackpad apparatus(device) 160, such as those described herein, may be used to facilitate interaction with the corresponding device.

The computing device 140 shown in FIG.9B includes a display device 150, and a combined keyboard and cardiovascular health monitoring trackpad apparatus 160.

As indicated in FIG. 9B, the display device 150 may be configured to render a GUI that allows a user to interact with the computing device 140, such as to run programs, browse the Internet or World Wide Web, or draft documents, as some examples. A user of the computing device 140 may interact with the computing device 140 via the GUI rendered on the display device 150 using the combined keyboard and cardiovascular health monitoring trackpad 160. For instance, the user may use the keyboard and trackpad 160 both to enter text or commands and for actions such as moving a cursor, selecting objects, launching programs from icons or moving objects in the GUI, as some examples. The keyboard and trackpad 160 may take a number of forms, and the particular arrangement of the keyboard and trackpad 160 will depend on the particular implementation. The keyboard and trackpad 160 may be implemented in a number of ways, such as using the techniques described herein, for example.

It will be appreciated that the particular configuration of the keyboard and trackpad 160 may vary and the configuration used will depend on the specific implementation. For instance, keyboard and trackpad 160 may be configured to function as both the keyboard and the trackpad and the keyboard and trackpad 160 may be configured to distinguish between keyboard actions and trackpad actions.

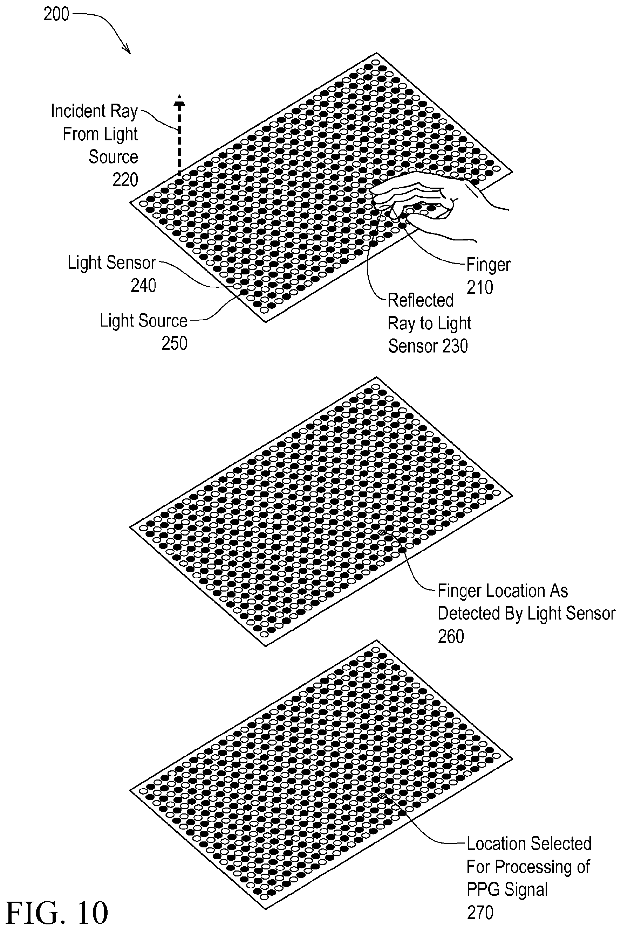

FIG.10 is a diagram illustrating the operation of a cardiovascular health monitoring trackpad apparatus in accordance with an example implementation. As illustrated, when the finger 210 is placed above the trackpad 200, the incident beam (ray) emitted by the light source 250 at the area where the finger 210 is placed is reflected by the finger 210 and sent to the light sensor 240 where it is received as reflected beam (ray) 230. The intensity of the reflected beam(ray) 230 varies with the proximity of the finger 210 to the trackpad 200. This is one of the many methods that may be used to find finger position. The cardiovascular health monitoring trackpad 200 may be configured such that when no change is finger position is detected for a set amount of time, the finger location 260 is selected as a PPG signal source 270 and signal from this location is selected for signal processing and extraction of cardiovascular parameters until change in finger locations is detected.

The trackpad 200 may be implemented, for example, in the computing device 100 as the trackpad apparatus 130 and in the computing device 140 as the keyboard and trackpad apparatus 160. Of course, the trackpad 200 may be implemented in conjunction with other computing devices and the computing devices 100 and 140 may include cardiovascular health monitoring trackpads having other configurations.

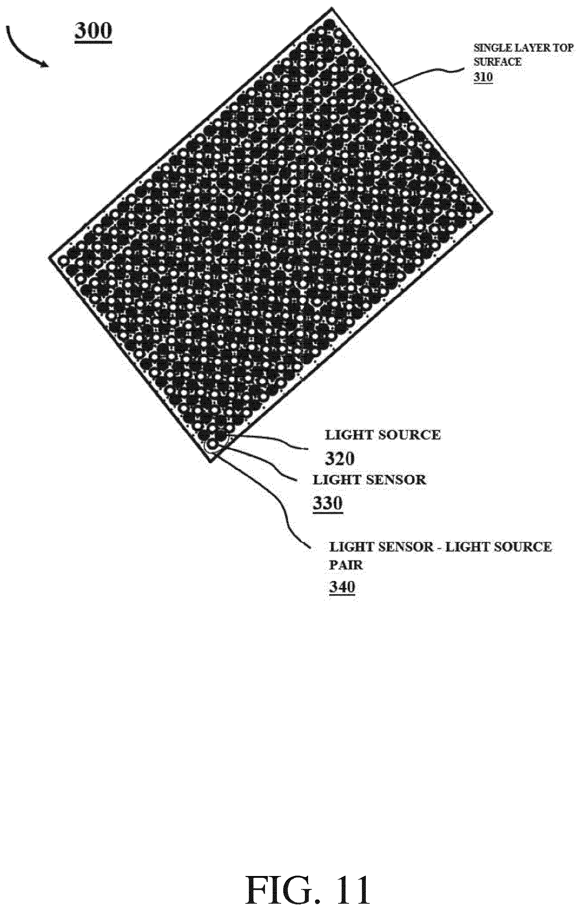

In trackpad 200, the light-sensor 240 and the light source 250 may be operably coupled to the top surface like illustrated in FIG.11 with trackpad 300.

Trackpad 300 may be implemented by placing an array (matrix) of light sensors and an array (matrix) of light sources on the entire area of the top surface. The two arrays may be arranged such that each cell of light sensor 330 has a cell of light source 320 to its adjacent, i.e. the cells of light sensor 330 and interleaved between the cells of lights source 320. Trackpad 300 may also be implemented by using an array of light-emitter/ detector pairs. The light sensor and the light source may be chosen such that they operate desirably at a matching range of wavelengths. Of course, the light sensor and light source may be implemented by other methods. It will be appreciated that the implementations discussed here are given by way of example and for purposes of illustration only.

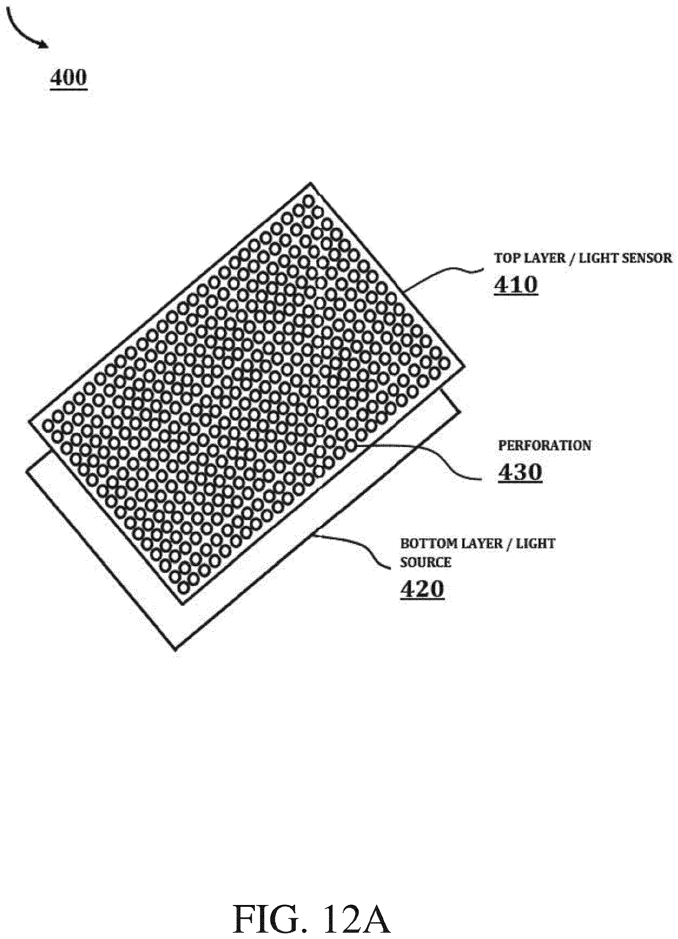

The operation illustrated by trackpad 200 may also be implemented as cardiovascular health monitoring trackpad with a double layer top surface like illustrated in FIG.12A with trackpad 400, whereby the light sensor 410 may be operably couple top layer and the top layer perforated 430. The light sensor may be implemented by using an array(matrix) of light sensors to occupy the entire area of the top layer except the perforations 430. The light source 420 may be coupled to the bottom layer (placed below the top layer) and incident rays (beams) from the light source may pass through the perforations 430.

The light source 420 may be implemented by backlighting or by using an array (matrix) of light emitting diodes (LEDs). The light sensor and the light source may be chosen such that they operate desirably at a matching range of wavelengths. Of course, the light sensor and light source may be implemented by other methods. It will be appreciated that the implementations discussed here are given by way of example and for purposes of illustration only.

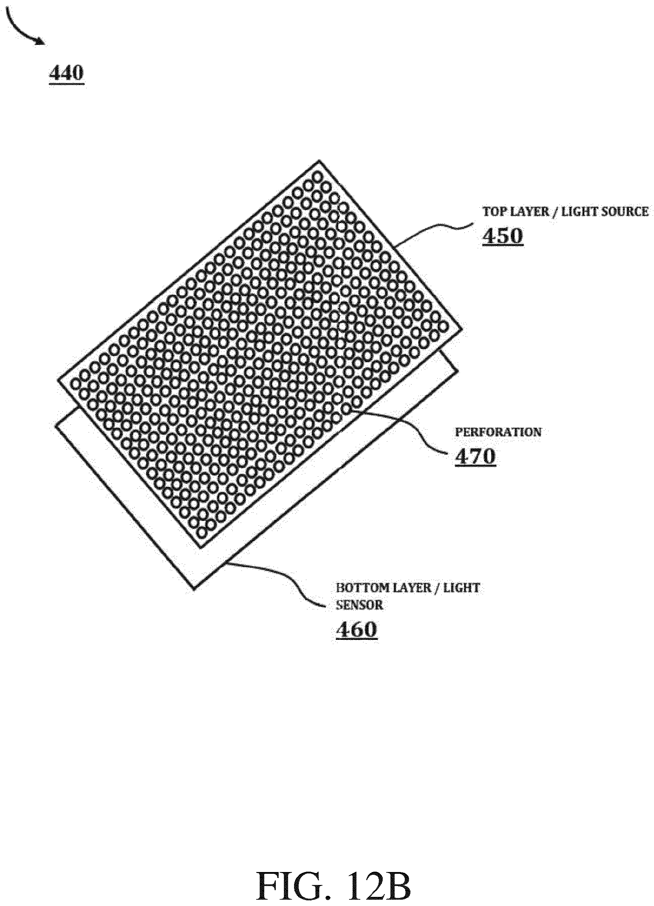

The operation illustrated with trackpad 200 may also be implemented as cardiovascular health monitoring trackpad with a double layer top surface by operably coupling the light source to the top layer and perforating the top layer like illustrated in FIG.12B with trackpad 440. The light source may be implemented by using an array (matrix) of light sources (e.g. LEDs) to occupy the entire area of the top layer except the perforations 470.

The light sensor 460 may be implemented by coupling an array (matrix)of light sensors to the bottom layer (placed below the top layer) such that the sensing area of the light sensors and the perforations are coincident. The light sensor 460 and the light source 450 may be chosen such that they operate desirably at a matching range of wavelengths. Of course, the light sensor and light source may be implemented by other methods. It will be appreciated that the implementations discussed here are given by way of example and for purposes of illustration only.

FIG.11 is a drawing illustrating a cardiovascular health monitoring trackpad in accordance with an example implementation. As indicated the cardiovascular health monitoring trackpad 300 comprises a light source 320 and a light sensor 330. The light sensor of the trackpad apparatus 300 may be a matrix of light sensors. The light source of the trackpad apparatus 300 may also be a matrix of light sources. The light sensor and light source of the trackpad apparatus may form a light-source/ light-sensor pair 340. These pairs may in turn form a matrix of light-source/light-sensor pairs. The light sensor and the light source may be chosen such that they operate desirably at a matching range of wavelengths. Of course, the light sensor and light source may be implemented by other methods. It will be appreciated that the implementations discussed here are given by way of example and for purposes of illustration only.

FIG. 12A is a drawing illustrating a cardiovascular health monitoring trackpad 400 with a double layered top surface in accordance with an example implementation. As indicated, the double layered trackpad 400 comprises a top layer with perforations 430 with the light sensor 410 operably coupled to the top layer and the light source 420 operably coupled to the bottom layer. The light source 420 may be implemented by backlighting or by using an array (matrix) of light sources. The light sensor may be implemented by using a matrix of light sensors to occupy the entire top layer except the perforations 430. The light sensor and the light source may be chosen such that they operate desirably at a matching range of wavelengths. Of course, the light sensor and light source may be implemented by other methods. It will be appreciated that the implementations discussed here are given by way of example and for purposes of illustration only.

FIG.12B is a drawing illustrating a cardiovascular health monitoring trackpad 440 with a double layered top surface in accordance with an example implementation. As indicated, the doubled layered trackpad 440 comprises a top layer with perforations 470 with the light source operably coupled to the top layer 450 and the light sensor operably coupled to the bottom layer 460.

The light sensor 460 may be implemented by coupling an array (matrix)of light sensors to the bottom layer (placed below the top layer) such that the sensing area of the light sensors and the perforations 470 are coincident. The light sensor 460 and the light source 450 may be chosen such that they operate desirably at a matching range of wavelengths. Of course, the light sensor and light source may be implemented by other methods. It will be appreciated that the implementations discussed here are given by way of example and for purposes of illustration only.

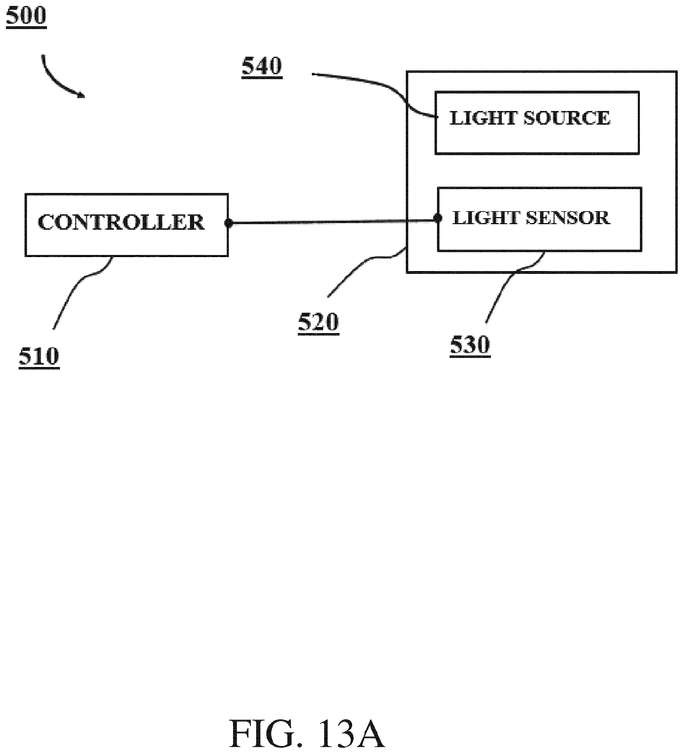

FIG.13A is a block diagram illustrating the cardiovascular health monitoring trackpad apparatus 500 in accordance with an example implementation. As indicated, the cardiovascular health monitoring trackpad apparatus includes a controller 510 and a cardiovascular health monitoring trackpad surface 520.

In the cardiovascular health monitoring trackpad apparatus 500, the light sensor of the cardiovascular health monitoring trackpad surface 530 is connected to the controller 510.The light source me be driven by other parts of the system (e.g. a different controller) or powered by other digital or analog means. The controller 510 and the light sensor 530 may be configured to select the location or locations of a finger (fingers) on the cardiovascular health monitoring trackpad surface 520 for continuous processing of PPG signal from that location (locations).

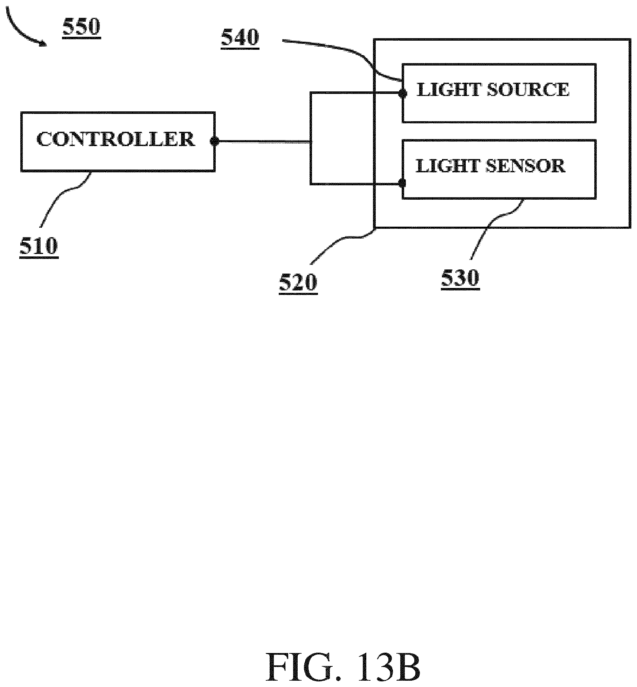

FIG.13B is a block diagram illustrating the cardiovascular health monitoring trackpad apparatus 550 in accordance with an example implementation. As indicated, the cardiovascular health monitoring trackpad apparatus 550 includes a controller 510 and a cardiovascular health monitoring trackpad surface 520.

In the cardiovascular health monitoring trackpad apparatus 550, the light sensor 530 and the light source 540 of the cardiovascular health monitoring trackpad surface a connected to the controller 510. The controller 510 may be configured to drive the light source of the cardiovascular health monitoring trackpad by means of applying direct current or by means of pulse width modulation (PWM). The controller 510 and the light sensor 530 may be configured to select the location or locations of a finger (fingers) on the cardiovascular health monitoring trackpad surface 520 for continuous processing of PPG signal from that location.

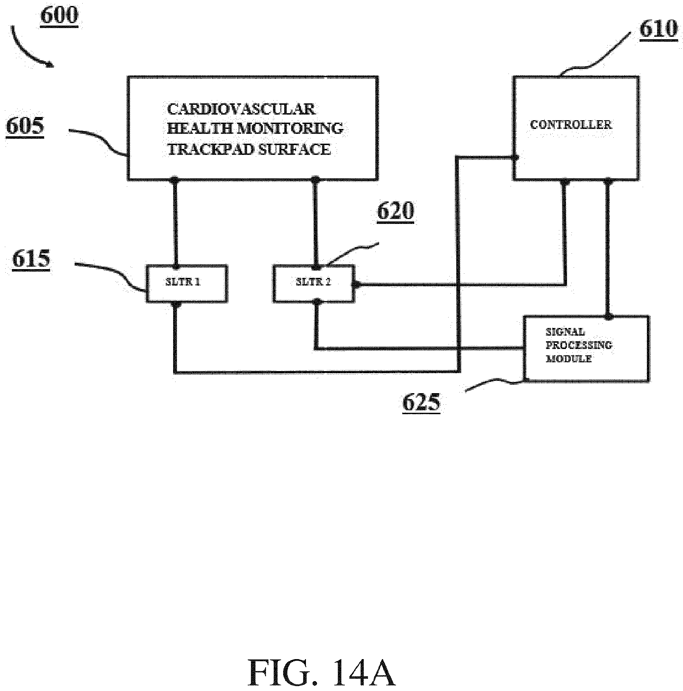

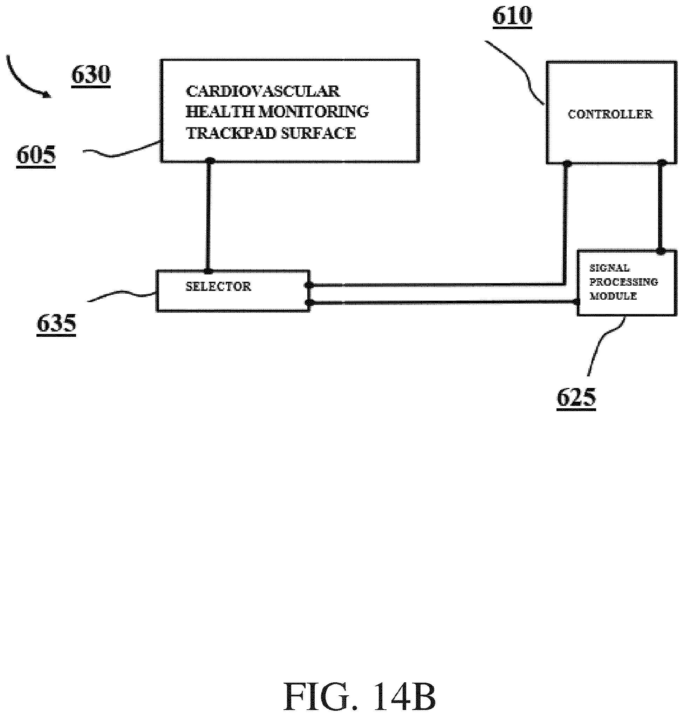

FIG.14A is a block diagram illustrating the cardiovascular health monitoring trackpad apparatus 600 in accordance with an example implementation. The cardiovascular health monitoring trackpad 600 can be implemented in single layer top surface form like cardiovascular health monitoring trackpad 300 or in a double layer top surface form like cardiovascular health monitoring trackpads 400 and 440. FIG.14A illustrates a cardiovascular health monitoring trackpad apparatus with two selectors, SLTR1 615 and SLTR2 620, a single signal processing module 625, a single controller 610 and a cardiovascular health monitoring trackpad surface 605. Of course the cardiovascular health monitoring trackpad apparatus may be Implemented in conjunction with other modules. For example, in other implementations only one selector may be used. In yet another implementation the functions of the selector and the signal processing module may be performed by the controller. For instance, as discussed in FIG.14B.

In certain implementations the cardiovascular health monitoring trackpad apparatus may include other elements or may be arranged in different fashions. In other instances, the signal processing module 625 may be included in the controller 610. In another instance, SLTR1 615 and SLTR2 620 may be included in the controller 610. In still another implementation, the functions of the signal processing module 625 may be performed by other elements of the computing system (e.g other than the controller 610) in which the cardiovascular health monitoring trackpad apparatus 600 is implemented. The functions of the SLTR1 660 and SLTR2 670 may also be performed by other parts of the system (e.g other than the controller 610) in which the cardiovascular health monitoring trackpad apparatus 600 is implemented.

In an example implementation of the cardiovascular health monitoring trackpad apparatus 600, the light source of the cardiovascular health monitoring trackpad surface 605 may be turned on initially and may be used with the light sensor to form a proximity sensor such that the incident ray (beam ) emitted by the light source at the area where a finger is located is reflected by the finger and received by the light sensor. This may be used to indicate (detect) the presence and location of a finger (i.e. a module for determining and optionally storing a location of a body part of a patient in relation to the array) on the cardiovascular health monitoring trackpad surface.

The SLTR1 615 and the controller 610 may store all locations of the cardiovascular health monitoring trackpad surface 605 where a finger is located (detected). A finger may also be located (detected) by reading the changes in the amount of ambient light at all locations of the cardiovascular health monitoring trackpad surface 605 or by using proximity thresholding.

The SLTR2 620, signal processing module 625 and the controller 610 may use information from SLTR1 615 and the controller 610 to select the location or locations of the finger (fingers) for continuous processing of PPG signal from that location.

FIG.14B is a block diagram illustrating the cardiovascular health monitoring trackpad apparatus 630 in accordance with an example implementation.

The cardiovascular health monitoring trackpad apparatus 630 can be implemented in single layer top surface form like cardiovascular health monitoring trackpad 300 or in a double layer top surface form like cardiovascular health monitoring trackpads 400 and 440. FIG. 14B illustrates a cardiovascular health monitoring trackpad apparatus with a single selector 6350, a single signal processing module 625, a single controller 610 and a cardiovascular health monitoring trackpad surface 630. Of course the cardiovascular health monitoring trackpad apparatus may be Implemented in conjunction with other modules. It will be appreciated that the cardiovascular health monitoring trackpad surface here is shown by example and for the purpose of illustration.

In certain implementations the cardiovascular health monitoring trackpad apparatus may include other elements or may be arranged in different fashions. In other instances, the signal processing module 625 may be included in the controller 610. In another instance, selector 635 may be included in the controller 610. In still another implementation, the functions of the signal processing module 625 may be performed by other elements of the computing system (e.g. other than the controller 610) in which the cardiovascular health monitoring trackpad apparatus 630 is implemented. The function of the selector 635 may also be performed by other parts of the system (e.g. other than the controller 610) in which the cardiovascular health monitoring trackpad apparatus 630 is implemented.

In an example implementation of the cardiovascular health monitoring trackpad apparatus 630, the light source of the cardiovascular health monitoring trackpad surface 605 may be turned on initially and may be used with the light sensor to form a proximity sensor such that the incident ray (beam) emitted by the light source at the area where a finger is located is reflected by the finger and received by the light sensor. This may be used to indicate (detect) the presence and location of a finger on the cardiovascular health monitoring trackpad surface.

In another example implementation of the cardiovascular health monitoring trackpad apparatus 630, the controller 610 may be used to control the light source of the cardiovascular health monitoring trackpad surface. The controller may be configured to drive the light source of the cardiovascular health monitoring trackpad by means of applying direct current or by means of pulse width modulation (PWM). The light source of the cardiovascular health monitoring trackpad surface may be controlled by other methods as well, such as using a different controller or applying direct current from a different source or using other digital or analog means

The selector 635 and the controller 610 may store all locations of the cardiovascular health monitoring trackpad surface 605 where a finger is located (detected). A finger may also be located (detected) by reading the changes in the amount of ambient light at all locations of the cardiovascular health monitoring trackpad surface 605 or by using proximity thresholding.

The selector 635, signal processing module 625 and the controller 610 may use information from the selector 635 and the controller 610 to select the location or locations of the finger (fingers) for continuous processing of PPG signal from that location.

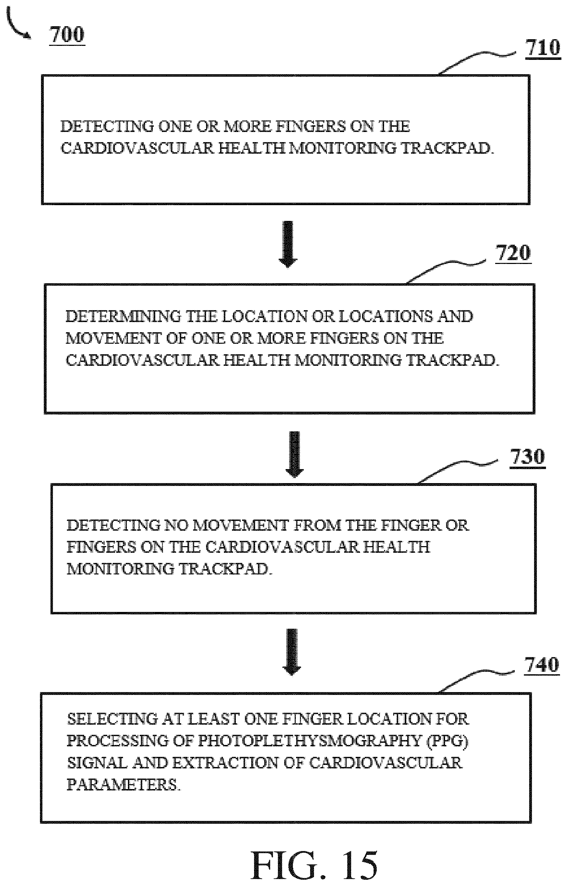

FIG.15 is a flow diagram illustrating the operation of cardiovascular health monitoring trackpad apparatus of FIGS. 10,11,12A,12B,13A,13B,14A and 14B. FIG 15 includes a process 700. The process includes detecting one or more fingers on the cardiovascular health monitoring trackpad 710. For example, the controller may be configured to use ambient light thresholding or proximity thresholding to detect one or more objects on the cardiovascular health monitoring trackpad surface. The process 700 further includes determining 720 the location or locations and movement of one or more fingers on the cardiovascular health monitoring trackpad, and detecting 730 no movement from the finger or fingers on the cardiovascular monitoring trackpad.

Process 700 ends with selecting at least one finger location for processing of photoplethysmography (PPG) signal and extraction of cardiovascular parameters from that location (where no movement is detected).

The controller of the cardiovascular surface apparatus described in this document may be implemented in a number of ways. The controller may be implemented using a general purpose programmable processor or controller. In other implementations, using an application specific integrated circuit which may include at least one processor. The controller may be implemented using a firmware/software in the form of machine readable instructions that may be executed by a general purpose processor or controller. The controller may also be implemented using a combination of techniques discussed above, or may be implemented using other techniques and/or devices.

The selector of the cardiovascular surface apparatus described in this document may be implemented in a number of ways. As an example, the selector may be implemented using mechanical switches, FETS or multiplexers.

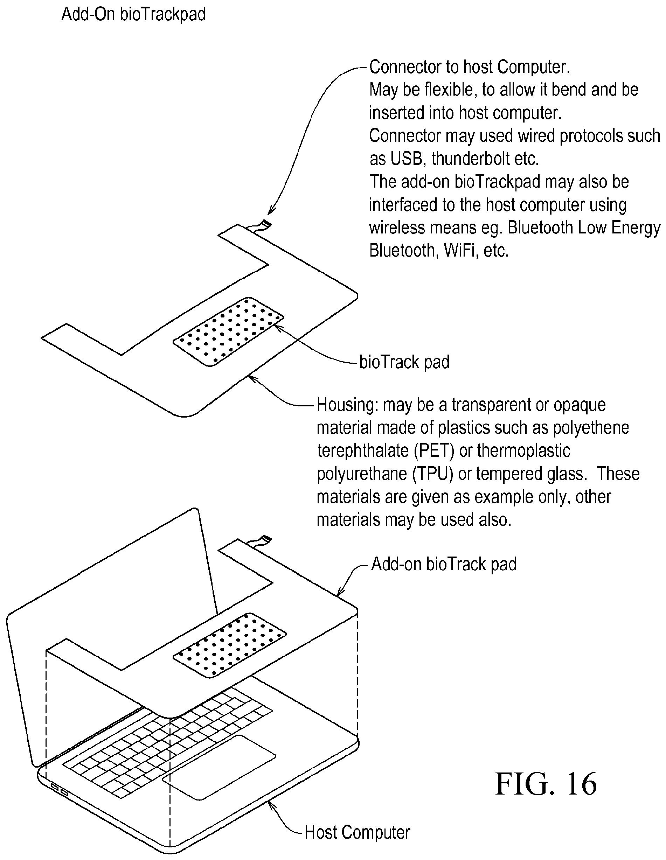

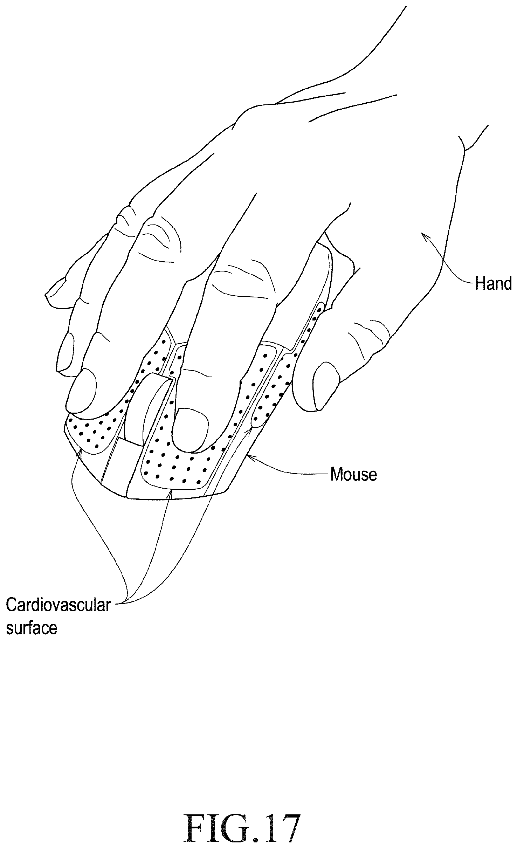

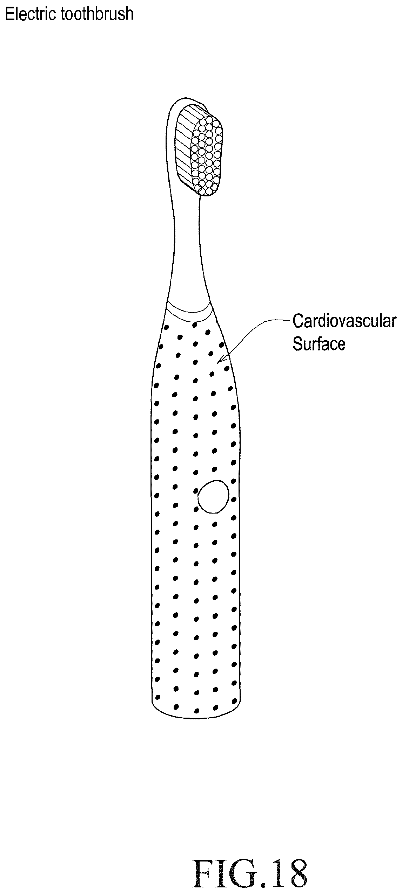

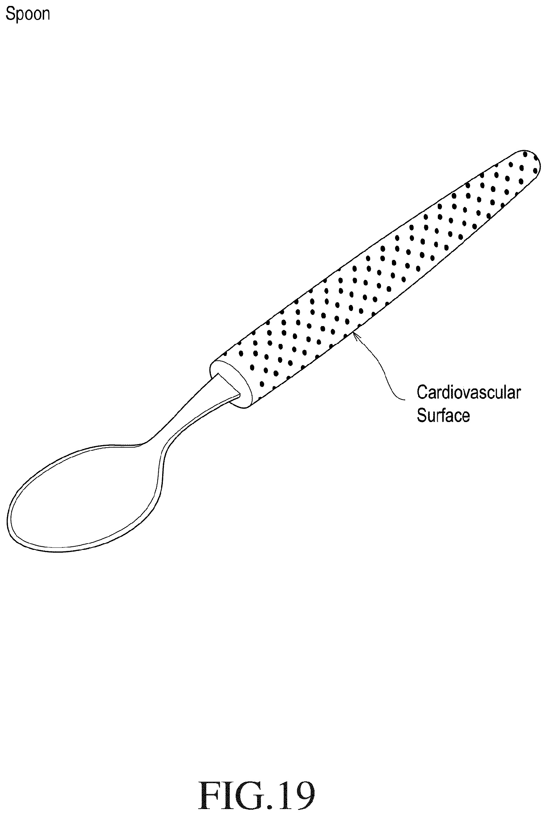

Figures 16 to 19 show examples of products incorporating the cardiovascular monitoring apparatus. In particular, Figure 16 shows the cardiovascular monitoring apparatus incorporated into a laptop trackpad, Figure 17 shows the cardiovascular monitoring apparatus incorporated into a mouse for a computer, Figure 18 shows the cardiovascular monitoring apparatus incorporated into the handle of an electric toothbrush, and Figure 19 shows the cardiovascular monitoring apparatus incorporated into the handle of a spoon.

As shown in Figure 16, the apparatus may be provided as an "add-on" to an existing product, where the apparatus generally fastens around or onto the product. In the particular example of Figure 16, the apparatus is provided as a layer which is attached onto a part of a laptop computer having a keyboard (where the layer does not extend over the keyboard). Generally, the sensing part of the apparatus may be provided such that it locates over the trackpad of the laptop computer, while the remainder of the layer is a housing formed of a material such as plastic. The apparatus may be configured to receive an input to control the laptop computer via the sensing part of the apparatus (i.e. the sensing part of the apparatus also operates as a conventional trackpad), and in this regard the layer may comprise a connector to the laptop computer (via a wired protocol such as USB). Alternatively, a wireless connection may be used.

Ideally, the products into which the cardiovascular surface is incorporated are products which tend to commonly be in contact with a patient. More ideally, the cardiovascular surface is incorporated into multiple products which tend to be used by the patient at different times of the day. For example, the electric toothbrush may be used by the patient early in the morning and late in the evening, the mouse for a computer and the trackpad may be used by the patient during the day while he or she is working, and the spoon may be used in the evening. Combining the cardiovascular information derived from each of the monitoring apparatuses throughout a day means that a more complete picture of the cardiovascular health of the patient is gained. Any portion of the apparatus and/or methods described herein may be combined in any combination, except mutually exclusive combination. The implementations described herein can include various combinations and/or sub-combinations of the functions, components and/or features of the different implementations described.

Reference numerals appearing in the claims are by way of illustration only and shall have no limiting effect on the scope of the claims.

## Claims

1. A cardiovascular monitoring apparatus, the apparatus comprising:
a photoplethysmography (PPG) sensor, having a plurality of light emitting elements (310) and a plurality of light sensing elements (320), for obtaining at least one PPG signal from a patient;
a member (300) for supporting the plurality of light emitting elements (310) and the plurality of light sensing elements (320), wherein the light emitting elements (310) and light sensing elements (320) are arranged as respective arrays on the member (300), and wherein the arrays are arranged such that the array of light emitting elements (310) are interleaved with the array of light sensing elements (320); and
a controller (530) comprising:
a detection module for detecting objects proximate the plurality of light sensing elements (320) thereby to determine a subset of the plurality of light sensing elements (320), and, subsequently to detecting objects, obtaining signals using the subset of the plurality of light sensing elements (320);
a determination module (550) for determining whether signals obtained by the subset of the plurality of light sensing elements (320) are each suitable for deriving information relevant to a patient's cardiovascular system; and
a processing module (540) for processing a signal that is determined to be suitable for deriving information relevant to the patient's cardiovascular system to derive information relevant to the patient's cardiovascular system.

2. An apparatus according to Claim 1, wherein, responsive to the determination module determining that a signal obtained by a particular light sensing element is not suitable for deriving information relevant to a patient's cardiovascular system, the determination module is arranged to determine whether a signal obtained by a different light sensing element is suitable for deriving information relevant to a patient's cardiovascular system.

3. An apparatus according to any preceding claim, wherein determining whether a signal obtained by a particular light sensing element is suitable for deriving information relevant to a patient's cardiovascular system comprises comparing the obtained signal against one or more criteria, preferably wherein the one or more criteria comprises at least one criterion relating to the shape of the signal waveform, more preferably wherein the comparing to the at least one criterion relating to the shape of the signal waveform is performed using at least one of: Cross-Correlation, Sign Comparison, Sum of Absolute Difference, Interval Matching, Dynamic Time Warping (DTW), and machine learning, and/or wherein the one or more criteria comprises at least one criterion relating to the frequency of the signal, and/or wherein the comparing comprises comparing at least one period of the signal, preferably exactly one period of the signal, to the one or more criteria, and/or wherein the apparatus further comprises means for storing a sample of the signal for comparison with the one or more criteria.

4. An apparatus according to Claim 3, wherein the controller further comprises a selection module for selecting at least one signal that is determined to be suitable for deriving information relevant to the patient's cardiovascular system for processing by the processing module; said selection being from a plurality of such signals, preferably wherein the selection is based on the comparison against the one or more criteria.

5. An apparatus according to any preceding claim, wherein the controller further comprises a detection module for detecting objects proximate the at least one light sensing element, preferably wherein detecting objects comprises detecting a parameter related to the ambient light received by the at least one light sensing element; and comparing the detected parameter to a threshold value.

6. An apparatus according to Claim 5, wherein the at least one light emitting element is adapted to emit light only when an object is detected proximate the at least one light sensing element and/or wherein the determination module is arranged to perform the determination on only those signals obtained by the at least one light sensing element in relation to which a proximate object is detected.

7. An apparatus according to any preceding claim, wherein the processing module terminates processing of the signal when the signal ceases to be suitable for deriving cardiovascular parameters, and/or wherein the apparatus further comprises a conditioner circuit, wherein the processing module processes the signal digitally.

8. An apparatus according to any preceding claim further comprising a module for determining (and optionally storing) at least one location within the array of the light sensing elements which obtains a signal that is determined to be suitable for deriving information relevant to a patient's cardiovascular system.

9. An apparatus according to any preceding claim, further comprising a module for determining movement of at least one body part of the patient in relation to the array; optionally wherein the at least one body part is at least one finger; preferably wherein the processing module is arranged to process only those signals that correspond to light sensing elements in relation to which no movement of the at least one body part is detected (more preferably over a predetermined period of time).

10. A cardiovascular monitoring apparatus according to any preceding claim, wherein at least one of the array of light emitting elements and the array of light sensing elements extend over an area of the member that is larger than 10 cm²; preferably larger than 15 cm²; and more preferably larger than 20 cm².

11. A cardiovascular monitoring apparatus according to any preceding claim, wherein the member is a flexible plastic substrate, and/or is curved.

12. A cardiovascular monitoring apparatus according to any preceding claim, wherein the array of light emitting elements is arranged in or on a first layer of the member and the array of light sensing elements is arranged in or on a second layer of the member, preferably wherein at least one of the first layer and the second layer comprises one or more apertures to allow light to pass therethrough, more preferably wherein one of the array of light emitting elements and the array of light sensing elements are formed of a light emitter or light sensor and the one or more apertures in the first layer or the second layer, wherein, in use, the light emitter or light sensor is provided underneath the layer having the one or more apertures, and yet more preferably wherein the other of the array of light emitting elements and the array of light sensing elements is arranged in or on the layer having the one or more apertures.

13. A consumer product incorporating the apparatus of any preceding claim, preferably wherein the at least one light sensing element is provided on a part of the product which is gripped by a user for using the product, more preferably wherein the plurality of light sensing elements are distributed across at least one surface of the product which is gripped by a user for using the product, still more preferably wherein the consumer product is: a trackpad for a computing device; or a computing device, such as a laptop computer, wherein the apparatus is provided on a user input device such as a trackpad.

14. A system comprising two or more separate cardiovascular monitoring apparatuses according to any of Claims 1 to 12, preferably wherein each of the separate cardiovascular monitoring apparatuses is connected to a network, and more preferably wherein the system further comprises computing means connected to the network adapted to store information obtained by each apparatus.

## Patentansprüche

1. Kardiovaskuläre Überwachungsvorrichtung, wobei die Vorrichtung umfasst:
einen Photoplethysmographiesensor (PPG-Sensor), aufweisend eine Vielzahl von lichtemittierenden Elementen (310) und eine Vielzahl von lichterfassenden Elementen (320), zum Beziehen mindestens eines PPG-Signals von einem Patienten;
ein Bauteil (300) zum Tragen der Vielzahl von lichtemittierenden Elementen (310) und der Vielzahl von lichterfassenden Elementen (320), wobei die lichtemittierenden Elemente (310) und die lichterfassenden Elemente (320) als jeweilige Arrays auf dem Bauteil (300) eingerichtet sind, und wobei die Arrays eingerichtet sind, so dass das Array von lichtemittierenden Elementen (310) mit dem Array von lichterfassenden Elementen (320) verschachtelt ist; und
einen Controller (530), umfassend:
ein Erkennungsmodul zum Erkennen von Objekten nahe der Vielzahl von lichterfassenden Elementen (320), wodurch ein Teilsatz der Vielzahl von lichterfassenden Elementen (320) bestimmt wird, und anschließend an das Erkennen von Objekten Beziehen von Signalen unter Verwendung des Teilsatzes der Vielzahl von lichterfassenden Elementen (320);
ein Bestimmungsmodul (550) zum Bestimmen, ob durch den Teilsatz der Vielzahl von lichterfassenden Elementen (320) bezogene Signale jeweils für ein Ableiten von Informationen geeignet sind, die für ein kardiovaskuläres System eines Patienten relevant sind; und
ein Verarbeitungsmodul (540) zum Verarbeiten eines Signals, von dem bestimmt wird, dass es für ein Ableiten von Informationen geeignet ist, die für das kardiovaskuläre System des Patienten relevant sind, um Informationen abzuleiten, die für das kardiovaskuläre System des Patienten relevant sind.

2. Vorrichtung nach Anspruch 1, wobei das Bestimmungsmodul als Reaktion darauf, dass das Bestimmungsmodul bestimmt, dass ein durch ein bestimmtes lichterfassendes Element bezogenes Signal nicht für ein Ableiten von Informationen geeignet sind, die für ein kardiovaskuläres System eines Patienten relevant sind, eingerichtet ist, um zu bestimmen, ob ein durch ein anderes lichterfassendes Element bezogenes Signal für ein Ableiten von Informationen geeignet ist, die für ein kardiovaskuläres System eines Patienten relevant sind.

3. Vorrichtung nach einem vorhergehenden Anspruch, wobei das Bestimmen, ob ein durch ein bestimmtes lichterfassendes Element bezogenes Signal für ein Ableiten von Informationen geeignet ist, die für ein kardiovaskuläres System eines Patienten relevant sind, ein Vergleichen des bezogenen Signals mit einem oder mehreren Kriterien umfasst, vorzugsweise wobei das eine oder die mehreren Kriterien mindestens ein Kriterium in Bezug auf die Form der Signalwellenform umfassen, mehr bevorzugt wobei das Vergleichen des mindestens einen Kriteriums in Bezug auf die Form der Signalwellenform unter Verwendung mindestens einer bzw. eines bzw. einem der folgenden durchgeführt wird: Kreuzkorrelation, Vorzeichenvergleich, Summe absoluter Differenzen, Intervallanpassung, Dynamic Time Warping (DTW) und maschinelles Lernen, und/oder wobei das eine oder die mehreren Kriterien mindestens ein Kriterium in Bezug auf die Frequenz des Signals umfassen, und/oder wobei das Vergleichen ein Vergleichen mindestens einer Periode des Signals, vorzugsweise exakt einer Periode des Signals mit dem einen oder den mehreren Kriterien umfasst, und/oder wobei die Vorrichtung ferner Mittel zum Speichern eines Abtastwerts des Signals zum Vergleich mit dem einen oder den mehreren Kriterien umfasst.

4. Vorrichtung nach Anspruch 3, wobei der Controller ferner ein Auswahlmodul zum Auswählen mindestens eines Signals, von dem bestimmt wird, dass es für ein Ableiten von Informationen geeignet ist, die für das kardiovaskuläre System des Patienten relevant sind, zum Verarbeiten durch das Verarbeitungsmodul umfasst; wobei die besagte Auswahl aus einer Vielzahl von derartigen Signalen ist, vorzugsweise wobei die Auswahl auf dem Vergleich mit dem einen oder den mehreren Kriterien basiert.

5. Vorrichtung nach einem vorhergehenden Anspruch, wobei der Controller ferner ein Erkennungsmodul zum Erkennen von Objekten nahe des mindestens einen lichterfassenden Elements umfasst, vorzugsweise wobei das Erkennen von Objekten ein Erkennen eines Parameters in Bezug auf das Umgebungslicht, das von dem mindestens einen lichterfassenden Element empfangen wird; und ein Vergleichen des erkannten Parameters mit einem Schwellenwert umfasst.

6. Vorrichtung nach Anspruch 5, wobei das mindestens eine lichtemittierende Element dazu angepasst ist, Licht nur zu emittieren, wenn ein Objekt nahe des mindestens einen lichterfassenden Elements erkannt wird, und/oder wobei das Bestimmungsmodul dazu eingerichtet ist, die Bestimmung an nur jenen Signalen durchzuführen, die durch das mindestens eine lichterfassende Element bezogen werden, in Bezug auf das ein nahes Objekt erkannt wird.

7. Vorrichtung nach einem vorhergehenden Anspruch, wobei das Verarbeitungsmodul eine Verarbeitung des Signals beendet, wenn das Signal aufhört, für ein Ableiten von kardiovaskulären Parametern geeignet zu sein, und/oder wobei die Vorrichtung ferner eine Konditionierschaltung umfasst, wobei das Verarbeitungsmodul das Signal digital verarbeitet.

8. Vorrichtung nach einem vorhergehenden Anspruch, ferner umfassend ein Modul zum Bestimmen (und optional Speichern) mindestens einer Stelle innerhalb des Arrays der lichterfassenden Elemente, das ein Signal bezieht, von dem bestimmt wird, dass es für ein Ableiten von Informationen geeignet ist, die für ein kardiovaskuläres System eines Patienten relevant sind.

9. Vorrichtung nach einem vorhergehenden Anspruch, ferner umfassend ein Modul zum Bestimmen einer Bewegung mindestens eines Körperteils des Patienten in Bezug auf das Array; optional wobei der mindestens eine Körperteil mindestens ein Finger ist; vorzugsweise wobei das Verarbeitungsmodul dazu eingerichtet ist, nur jene Signale zu verarbeiten, die lichterfassenden Elementen entsprechen, in Bezug auf die keine Bewegung des mindestens einen Körperteils erkannt wird (mehr bevorzugt über einen vorbestimmten Zeitraum).

10. Kardiovaskuläre Überwachungsvorrichtung nach einem vorhergehenden Anspruch, wobei sich mindestens eines von dem Array von lichtemittierenden Elementen und dem Array von lichterfassenden Elementen über eine Fläche des Bauteils erstreckt, die größer als 10 cm², vorzugsweise größer als 15 cm² und mehr bevorzugt größer als 20 cm² ist.

11. Kardiovaskuläre Überwachungsvorrichtung nach einem vorhergehenden Anspruch, wobei das Bauteil ein flexibles Kunststoffsubstrat ist und/oder gewölbt ist.

12. Kardiovaskuläre Überwachungsvorrichtung nach einem vorhergehenden Anspruch, wobei das Array von lichtemittierenden Elementen in oder auf einer ersten Schicht des Bauteils eingerichtet ist und das Array von lichterfassenden Elementen in oder auf einer zweiten Schicht des Bauteils eingerichtet ist, vorzugsweise wobei mindestens eine von der ersten Schicht und der zweiten Schicht eine oder mehrere Durchlässe umfasst, um zu ermöglichen, dass Licht dort hindurchtritt, mehr bevorzugt wobei eines von dem Array von lichtemittierenden Elementen und dem Array von lichterfassenden Elementen von einem Lichtemitter oder Lichtsensor und dem einen oder den mehreren Durchlässen in der ersten Schicht oder der zweiten Schicht gebildet wird, wobei der Lichtemitter oder Lichtsensor im Gebrauch unter der Schicht mit dem einen oder den mehreren Durchlässen vorgesehen ist, und noch mehr bevorzugt wobei das andere von dem Array von lichtemittierenden Elementen und dem Array von lichterfassenden Elementen in oder auf der Schicht mit dem einen oder den mehreren Durchlässen eingerichtet ist.

13. Verbraucherprodukt, einbindend die Vorrichtung nach einem vorhergehenden Anspruch, vorzugsweise wobei das mindestens eine lichterfassende Element auf einem Teil des Produkts vorgesehen ist, der von einem Benutzer zum Verwenden des Produkts ergriffen wird, mehr bevorzugt wobei die Vielzahl von lichterfassenden Elementen über mindestens eine Oberfläche des Produkts verteilt ist, die von einem Benutzer zum Verwenden des Produkts ergriffen wird, noch mehr bevorzugt wobei das Verbraucherprodukt ein Trackpad für ein Rechengerät; oder ein Rechengerät, wie ein Laptop-Computer, ist, wobei die Vorrichtung auf einem Benutzereingabegerät, wie einem Trackpad, vorgesehen ist.

14. System, umfassend zwei oder mehr separate kardiovaskuläre Überwachungsvorrichtungen nach einem der Ansprüche 1 bis 12, vorzugsweise wobei jede der separaten kardiovaskulären Überwachungsvorrichtungen mit einem Netzwerk verbunden ist, und mehr bevorzugt wobei das System ferner mit dem Netzwerk verbundene Rechenmittel umfasst, die dazu angepasst sind, Informationen zu speichern, die durch jede Vorrichtung bezogen werden.

## Revendications

1. Appareil de surveillance cardiovasculaire, l'appareil comprenant :
un capteur de photopléthysmographie (PPG), ayant une pluralité d'éléments d'émission de lumière (310) et une pluralité d'éléments de détection de lumière (320), destiné à obtenir au moins un signal PPG d'un patient ;
un membre (300) destiné à supporter la pluralité d'éléments d'émission de lumière (310) et la pluralité d'éléments de détection de lumière (320), dans lequel les éléments d'émission de lumière (310) et les éléments de détection de lumière (320) sont agencés comme des réseaux respectifs sur le membre (300), et dans lequel les réseaux sont agencés de sorte que le réseau d'éléments d'émission de lumière (310) est entrelacé avec le réseau d'éléments de détection de lumière (320) ; et
un dispositif de commande (530) comprenant :
un module de détection destiné à détecter des objets à proximité de la pluralité d'éléments de détection de lumière (320) afin de déterminer ainsi un sous-ensemble de la pluralité d'éléments de détection de lumière (320), et, suite à la détection d'objets, à obtenir des signaux en utilisant le sous-ensemble de la pluralité d'éléments de détection de lumière (320) ;
un module de détermination (550) destiné à déterminer si les signaux obtenus par le sous-ensemble de la pluralité d'éléments de détection de lumière (320) sont chacun appropriés pour dériver des informations pertinentes pour le système cardiovasculaire d'un patient; et
un module de traitement (540) destiné à traiter un signal qui est déterminé comme étant approprié pour dériver des informations pertinentes pour le système cardiovasculaire du patient afin de dériver des informations pertinentes pour le système cardiovasculaire du patient.

2. Appareil selon la revendication 1, dans lequel, en réponse à la détermination par le module de détermination qu'un signal obtenu par un élément de détection de lumière particulier n'est pas approprié pour dériver des informations pertinentes pour le système cardiovasculaire d'un patient, le module de détermination est agencé pour déterminer si un signal obtenu par un élément de détection de lumière différent est approprié pour dériver des informations pertinentes pour le système cardiovasculaire d'un patient.

3. Appareil selon l'une quelconque des revendications précédentes, dans lequel le fait de déterminer si un signal obtenu par un élément de détection de lumière particulier est approprié pour dériver des informations pertinentes pour le système cardiovasculaire d'un patient comprend la comparaison du signal obtenu avec un ou plusieurs critères, préférablement dans lequel les un ou plusieurs critères comprennent au moins un critère relatif à la forme d'onde de signal, plus préférablement dans lequel la comparaison de l'au moins un critère relatif à la forme d'onde de signal est effectuée à l'aide d'au moins un élément parmi : la corrélation croisée, la comparaison de signes, la somme des différences absolues, la mise en correspondance d'intervalles, la déformation temporelle dynamique (DTW), et l'apprentissage automatique, et/ou dans lequel les un ou plusieurs critères comprennent au moins un critère relatif à la fréquence du signal, et/ou dans lequel la comparaison comprend la comparaison d'au moins une période du signal, préférablement exactement une période du signal, à un ou plusieurs critères, et/ou dans lequel l'appareil comprend en outre des moyens de stockage d'un échantillon du signal pour la comparaison avec les un ou plusieurs critères.

4. Appareil selon la revendication 3, dans lequel le dispositif de commande comprend en outre un module de sélection destiné à sélectionner au moins un signal qui est déterminé comme étant approprié pour dériver des informations pertinentes pour le système cardiovasculaire du patient pour un traitement par le module de traitement ; ladite sélection se faisant à partir d'une pluralité de tels signaux, préférablement dans lequel la sélection est basée sur la comparaison avec les un ou plusieurs critères.

5. Appareil selon l'une quelconque des revendications précédentes, dans lequel le dispositif de commande comprend en outre un module de détection destiné à détecter des objets à proximité de l'au moins un élément de détection de lumière, préférablement dans lequel la détection d'objets comprend la détection d'un paramètre relatif à la lumière ambiante reçue par l'au moins un élément de détection de lumière ; et la comparaison du paramètre détecté à une valeur de seuil.

6. Appareil selon la revendication 5, dans lequel l'au moins un élément d'émission de lumière est conçu pour émettre de la lumière uniquement lorsqu'un objet est détecté à proximité de l'au moins un élément de détection de lumière et/ou dans lequel le module de détermination est agencé pour effectuer la détermination uniquement sur ces signaux obtenus par l'au moins un élément de détection de lumière par rapport auxquels un objet proche est détecté.

7. Appareil selon l'une quelconque des revendications précédentes, dans lequel le module de traitement termine le traitement du signal lorsque le signal cesse d'être approprié pour dériver des paramètres cardiovasculaires, et/ou dans lequel l'appareil comprend en outre un circuit conditionneur, dans lequel le module de traitement traite le signal numériquement.

8. Appareil selon l'une quelconque des revendications précédentes, comprenant en outre un module destiné à déterminer (et facultativement stocker) au moins un emplacement au sein du réseau des éléments de détection de lumière qui obtient un signal qui est déterminé comme étant approprié pour dériver des informations pertinentes pour le système cardiovasculaire d'un patient.

9. Appareil selon l'une quelconque des revendications précédentes, comprenant en outre un module destiné à déterminer le mouvement d'au moins une partie de corps du patient par rapport au réseau ; facultativement dans lequel l'au moins une partie de corps est au moins un doigt ; préférablement dans lequel le module de traitement est agencé pour traiter uniquement ces signaux qui correspondent à des éléments de détection de lumière par rapport auxquels aucun mouvement de l'au moins une partie de corps n'est détecté (plus préférablement sur une période de temps prédéterminée).

10. Appareil de surveillance cardiovasculaire selon l'une quelconque des revendications précédentes, dans lequel au moins l'un du réseau d'éléments d'émission de lumière et du réseau d'éléments de détection de lumière s'étend sur une zone du membre qui est supérieure à 10 cm² ; préférablement supérieure à 15 cm² ; et plus préférablement supérieure à 20 cm².

11. Appareil de surveillance cardiovasculaire selon l'une quelconque des revendications précédentes, dans lequel le membre est un substrat en plastique flexible, et/ou est incurvé.

12. Appareil de surveillance cardiovasculaire selon l'une quelconque des revendications précédentes, dans lequel le réseau d'éléments d'émission de lumière est agencé dans ou sur une première couche du membre et le réseau d'éléments de détection de lumière est agencé dans ou sur une deuxième couche du membre, préférablement dans lequel au moins l'une de la première couche et de la deuxième couche comprend une ou plusieurs ouvertures pour permettre à la lumière de passer à travers, plus préférablement dans lequel l'un du réseau d'éléments d'émission de lumière et du réseau d'éléments de détection de lumière est formé d'un émetteur de lumière ou d'un capteur de lumière et des unes ou plusieurs ouvertures dans la première couche ou la deuxième couche, dans lequel, en cours d'utilisation, l'émetteur de lumière ou le capteur de lumière est disposé sous la couche ayant les une ou plusieurs ouvertures, et encore plus préférablement dans lequel l'autre du réseau d'éléments d'émission de lumière et du réseau d'éléments de détection de lumière est agencé dans ou sur la couche ayant les une ou plusieurs ouvertures.

13. Produit de consommation incorporant l'appareil selon l'une quelconque des revendications précédentes, préférablement dans lequel l'au moins un élément de détection de lumière est fourni sur une partie du produit qui est saisie par un utilisateur pour utiliser le produit, plus préférablement dans lequel la pluralité d'éléments de détection de lumière sont distribués sur au moins une surface du produit qui est saisie par un utilisateur pour utiliser le produit, encore plus préférablement dans lequel le produit de consommation est : un pavé tactile pour un dispositif informatique ; ou un dispositif informatique, tel qu'un ordinateur portable, dans lequel l'appareil est fourni sur un dispositif d'entrée d'utilisateur tel qu'un pavé tactile.

14. Système comprenant deux appareils de surveillance cardiovasculaire distincts ou plus selon l'une quelconque des revendications 1 à 12, préférablement dans lequel chacun des appareils de surveillance cardiovasculaire distincts est connecté à un réseau, et plus préférablement dans lequel le système comprend en outre des moyens informatiques connectés au réseau conçus pour stocker des informations obtenues par chaque appareil.
